# EUROPEAN PATENT APPLICATION

(11) **EP 1 361 219 A1**
(43) Date of publication of application: **12.11.2003**
(21) Application number: 02010643.1
(22) Date of filing: 11.05.2002
(51) Int. Cl.: C07D 251/18, A01N 43/68

(54) **Herbicidal indanylalkylamino-1,3,5-triazines, process for their preparation, compositions thereof, and their use as herbicides and plant growth regulators**

(71) Applicant: Bayer CropScience GmbH, 65929 Frankfurt/Main (DE)
(72) Inventor: Angermann, Alfred Dr., 65830 Kriftel (DE); Giencke, Wolfgang Dr., 65719 Hofheim (DE); Dietrich, Hansjörg Dr., 65719 Hofheim (DE); Auler, Thomas Dr., 65812 Bad Soden (DE); Menne, Hubert Dr., 65719 Hofheim (DE); Bieringer, Hermann Dr., 65817 Eppstein (DE)

(57) **Abstract**

The invention relates to indanylalkylamino-1,3,5-triazine derivative of formula (I) or a salt thereof: 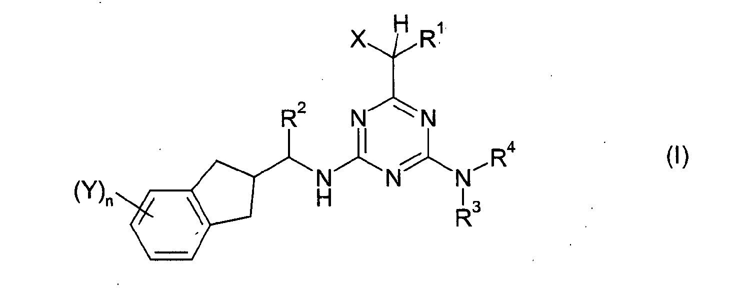 wherein the various symbols are as defined in claim 1, process for their preparation, compositions thereof, and their use as herbicides or plant growth regulators.

## Description

The invention relates to 2-amino-4-(substituted alkyl)-6-[1-(indan-2-yl)-alkylamino]-1,3,5-triazine derivatives, processes for their preparation, to compositions thereof, and to their use as herbicides or plant growth regulators.

### Background of the invention

WO 97/29095 describes the preparation of 2,4-diamino-1,3,5-triazines and their use as herbicides and plant growth regulators. In this reference certain 2-amino-6-[1-(indanyl)-1-alkylamino]-1,3,5-triazine derivatives are disclosed as herbicides.
In some cases, the known active substances have disadvantages when used, for example an insufficient herbicidal action against harmful plants, too rigid applicational limitations related to weather, climate and/or soil conditions, too narrow a spectrum against weeds or too little crop selectivity.
It has now been found that specific substituted 2-amino-4-(substituted alkyl)-6-[1-(indan-2-yl)-alkylamino]-1,3,5-triazine derivatives possess advantageous applicational properties compared with the prior art compounds. For instance they are highly effective selective herbicides which can be used for the control of a range of harmful weeds in crops of useful plants. They exhibit a remarkable herbicidal efficacy at a high level of crop safety.

### Detailed Description of the Invention

The present invention relates to a compound which is an indanylalkylamino-1,3,5-triazine derivative of formula (I): 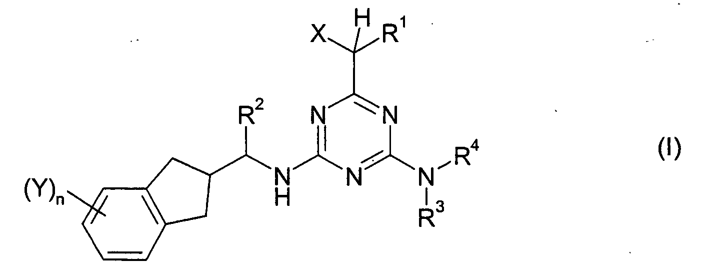 in which:
R¹ is (C₁-C₆)alkyl, (C₁-C₆)haloalkyl, [(C₁-C₄)alkoxy](C₁-C₆)alkyl, (C₂-C₆)alkenyl, (C₂-C₆)haloalkenyl, (C₄-C₆)cycloalkenyl, (C₄-C₆)halocycloalkenyl, (C₁-C₆)alkoxy or (C₁-C₆)haloalkoxy; or is (C₃-C₆)cycloalkyl or (C₃-C₆)halocycloalkyl which cycloalkyl groups are unsubstituted or substituted by one or two (C₁-C₄)alkyl groups;
R² is (C₁-C₄)alkyl, (C₁-C₄)haloalkyl, (C₃-C₆)cycloalkyl or (C₃-C₆)halocycloalkyl;
X is halogen or OH;
R³ and R⁴ are each independently H, (C₁-C₄)alkyl, (C₁-C₄)haloalkyl, (C₃-C₄)alkenyl, (C₃-C₄)haloalkenyl, (C₃-C₄)alkynyl, (C₃-C₄)haloalkynyl or an acyl radical; or
R³ and R⁴ together with the attached nitrogen atom form a five, six or seven membered saturated heterocyclic ring in which one of the ring carbon atoms may be replaced by a hetero atom selected from N, O and S, and which ring is unsubstituted or substituted by (C₁-C₄)alkyl (for example a pyrrolidine, piperidine or morpholine ring); and
(Y)ₙ is n radicals Y where each Y is independently (C₁-C₄)alkyl, preferably (C₁-C₃)alkyl such as methyl, ethyl, n-propyl, iso-propyl; or (C₁-C₃)haloalkyl, halogen, (C₁-C₃)alkoxy, (C₁-C₃)haloalkoxy or CN; and
n is 0, 1, 2, 3 or 4 (preferably n is 0, 1 or 2);
or an agriculturally acceptable salt thereof.

The subject matter of the invention is also directed to all stereoisomers which are encompassed by formula (I), and their mixtures. Such compounds of the formula (I) contain at least two or more asymmetric carbon atoms or else double bonds which are not stated specifically in the formula (I). It will be understood that the present invention embraces both the pure isomers and more or less enriched mixtures thereof as well as the racemic mixtures.
The possible stereoisomers which are defined by their specific spatial form, such as enantiomers, diastereomers, Z- and E-isomers, are all encompassed by formula (I) and can be obtained by customary methods from mixtures of the stereoisomers, or else be prepared by stereoselective reactions in combination with the use of stereochemically pure starting materials.
The two centres of asymmetry referred to above are the carbon atoms adjacent to R¹ and to R². They are both centres of asymmetry, so that the compound can exist as at least four stereoisomeric forms, each asymmetric carbon atom having (*R*) or (*S*) configuration according to the Cahn-Ingold-Prelog system. Moreover depending on the nature of the groups R¹, R², R³, R⁴ and (Y)ₙ, further asymmetric carbon atoms may be present, for example when the group (Y)ₙ results in an unsymmetrically substituted indanyl ring in formula (I), then the indanyl ring carbon atom which is bonded to the other portion of the structure is also a centre of asymmetry.
The invention also encompasses any keto and enol tautomer forms and mixtures thereof.

The compounds of the formula (I) can form salts by addition of a suitable inorganic or organic acid such as, for example, HCI, HBr, H₂SO₄ or HNO₃, or a mono- or bifunctional carboxylic acid or sulfonic acid, to a basic group such as, for example, amino or alkylamino. Suitable substituents which are present in deprotonated form such as, for example, sulfonic acids or carboxylic acids, can form internal salts with groups which are protonable themselves, such as amino groups. Likewise, salts can be formed by replacing the hydrogen in suitable substituents such as, for example, sulfonic acids or carboxylic acids, by an agriculturally suitable cation. These salts are, for example, metal salts, in particular alkali metal salts or alkaline earth metal salts, in particular sodium salts and potassium salts, or else ammonium salts, salts with organic amines, or quaternary ammonium salts.

In the present patent specification, including the accompanying claims, the aforementioned substituents have the following meanings:
Halogen means fluorine, chlorine, bromine or iodine.
The term "halo" before the name of a radical means that this radical is partially or completely halogenated, that is to say, substituted by F, Cl, Br, or I, in any combination.
The expression "(C₁-C₆)alkyl" means an unbranched or branched non-cyclic saturated hydrocarbon radical having 1, 2, 3, 4, 5 or 6 carbon atoms (indicated by a range of C-atoms in the parenthesis), such as, for example a methyl, ethyl, propyl, isopropyl, 1-butyl, 2-butyl, 2-methylpropyl or tert-butyl radical. The same applies to alkyl groups in composite radicals such as "alkoxyalkyl".
Alkyl radicals and also in composite groups, unless otherwise defined, preferably have 1 to 4 carbon atoms.
"(C₁-C₆)Haloalkyl" means an alkyl group mentioned under the expression
"(C₁-C₆)alkyl" in which one or more hydrogen atoms are replaced by the same number of identical or different halogen atoms, such as monohaloalkyl, perhaloalkyl, CF₃, CHF₂, CH₂F, CHFCH₃, CF₃CH₂, CF₃CF₂, CHF₂CF₂, CH₂FCHCl, CH₂Cl, CCl₃, CHCl₂ or CH₂CH₂Cl.
"[(C₁-C₄)alkoxy](C₁-C₆)alkyl" means (C₁-C₆)alkyl which is substituted by (C₁-C₄)alkoxy.
"(C₁-C₆)Alkoxy" means an alkoxy group whose carbon chain has the meaning given under the expression "(C₁-C₆)alkyl". "Haloalkoxy" is, for example, OCF₃, OCHF₂, OCH₂F, CF₃CF₂O, OCH₂CF₃ or OCH₂CH₂Cl.
"(C₂-C₆)Alkenyl" means an unbranched or branched non-cyclic carbon chain having a number of carbon atoms which corresponds to this stated range and which contains at least one double bond which can be located in any position of the respective unsaturated radical. "(C₂-C₆)Alkenyl" accordingly denotes, for example, the vinyl, allyl, 2-methyl-2-propenyl, 2-butenyl, pentenyl, 2-methylpentenyl or the hexenyl group.
"(C₃-C₄)Alkynyl" means an unbranched or branched non-cyclic carbon chain having a number of carbon atoms which corresponds to this stated range and which contains one triple bond which can be located in any position of the respective unsaturated radical. "(C₃-C₄)Alkynyl" accordingly denotes, for example, the propargyl, 1-methyl-2-propynyl, 2-butynyl or 3-butynyl group.
"(C₃-C₆)Cycloalkyl" denotes monocyclic alkyl radicals, such as the cyclopropyl, cyclobutyl, cyclopentyl or cyclohexyl radical.
"(C₄-C₆)Cycloalkenyl" denotes a carbocyclic, nonaromatic, partially unsaturated ring having 4 to 6 carbon atoms, for example 1-cyclobutenyl, 2-cyclobutenyl, 1-cyclopentenyl, 2-cyclopentenyl, 3-cyclopentenyl, or 1-cyclohexenyl, 2-cyclohexenyl, 3-cyclohexenyl, 1,3-cyclohexadienyl or 1,4-cyclohexadienyl.

An acyl radical is, in a broad sense, the radical of an organic acid which is formed formally by removing an OH group, for example the radical of a carboxylic acid and radicals of acids derived therefrom, such as thiocarboxylic acid, unsubstituted or N-substituted iminocarboxylic acids or the radical of carbonic monoesters, unsubstituted or N-substituted carbamic acid, sulfonic acids, sulfinic acids, phosphonic acids, and phosphinic acids. Acyl is, for example, formyl, alkylcarbonyl such as [(C₁-C₄)alkyl]carbonyl, phenylcarbonyl, alkyloxycarbonyl, phenyloxycarbonyl; benzyloxycarbonyl, alkylsulfonyl, alkylsulfinyl, N-alkyl-1-iminoalkyl and other radicals of organic acids. In this context, the radicals can be even further substituted in each of the alkyl or phenyl moieties, for example in the alkyl moiety by one or more radicals selected from the group consisting of halogen, alkoxy, phenyl and phenoxy; examples of substituents in the phenyl moiety are mono- or polysubstituted, preferably up to trisubstituted, identical or different radicals selected from the group consisting of halogen, (C₁-C₄)alkyl, (C₁-C₄)alkoxy, (C₁-C₄)haloalkyl, (C₁-C₄)haloalkoxy and nitro, for example o-, m- and p-tolyl, dimethylphenyls, 2-, 3-and 4-chlorophenyl, 2-, 3- and 4-trifluoro- and -trichlorophenyl, 2,4-, 3,5-, 2,5- and 2,3-dichlorophenyl, o-, m- and p-methoxyphenyl.
The acyl radical generally has one to 24 carbon atoms, preferably 1 to 18, more preferably 1 to 12, most preferably 1 to 6, in particular 1 to 4.
Acyl in the narrower sense is, for example, the radical of an alkanoic acid, alkenoic acid, alkynoic acid, arylcarboxylic acid (for example benzoyl), alkoxycarbonyl, alkenyloxycarbonyl, alkynyloxycarbonyl, aryloxycarbonyl, alkylsulfonyl or alkysulfinyl; in an even narrower sense, acyl is a radical of an alkanoic acid, for example a (C₁-C₂₄)alkanoic acid, preferably (C₁-C₁₈)alkanoic acid, in particular (C₁-C₁₂)alkanoic acid, very especially (C₁-C₆)alkanoic acid such as formyl, acetyl or propionyl.

The expression "one or more radicals selected from the group consisting of" in the definition is to be understood as meaning in each case one or more identical or different radicals selected from the stated group of radicals, unless specific limitations are defined expressly.

By combination of variables the generic formulae may formally define unstable functional groups, e.g. the carbamyl radical or the hydroxy carbonyloxy radical, which are unstable in neutral or acidic aqueous medium and which thus are not preferred or are used by way of their stable salts or degradation products only, respectively.

Compounds of the stated formula (I) according to the invention or their salts in which individual radicals have one of the preferred meanings which have already been stated or are stated hereinbelow, or in particular those in which one or more of the preferred meanings which have already been stated or which are stated hereinbelow are combined, are of particular interest, mainly because of the more potent herbicidal action, better selectivity and/or greater ease of preparation.

Preferably R¹ is (C₁-C₄)alkyl, such as methyl, ethyl, n-propyl or iso-propyl; or (C₁-C₃)haloalkyl, [(C₁-C₂)alkoxy](C₁-C₃)alkyl, (C₂-C₃)alkenyl, (C₂-C₃)haloalkenyl, (C₅-C₆)cycloalkenyl, (C₅-C₆)halocycloalkenyl, (C₁-C₃)alkoxy or (C₁-C₃)haloalkoxy; or is (C₃-C₅)cycloalkyl or (C₃-C₅)halocycloalkyl which cycloalkyl groups are unsubstituted or substituted by one or two methyl or ethyl groups;
more preferably R¹ is (C₁-C₃)alkyl; most preferably R¹ is (C₁-C₂)alkyl.

Independently of the radicals R¹, R³, R⁴, X and (Y)ₙ and preferably in combination with preferred meanings of one or more of these radicals, the following meanings of R² are of particular interest.
Preferably R² is (C₁-C₄)alkyl, (C₁-C₃)haloalkyl, (C₃-C₅)cycloalkyl or (C₃-C₅)halocycloalkyl;
more preferably R² is (C₁-C₄)alkyl or (C₃-C₅)cycloalkyl; most preferably R² is (C₁-C₃)alkyl or (C₃-C₅)cycloalkyl.

Independently of the radicals R¹, R², R³, R⁴ and (Y)ₙ and preferably in combination with preferred meanings of one or more of these radicals, the following meanings of X are of particular interest.
Preferably X is Cl, F or OH; more preferably X is Cl or F.

Independently of the radicals R¹, R², R⁴, X and (Y)ₙ and preferably in combination with preferred meanings of one or more of these radicals, the following meanings of R³ are of particular interest.
Preferably R³ is H, (C₁-C₄)alkyl, (C₁-C₄)haloalkyl, (C₃-C₄)alkenyl, (C₃-C₄)alkynyl or an acyl radical having 1 to 12 carbon atoms (preferably CHO, -CO(C₁-C₆)alkyl, -CO(C₁-C₆)haloalkyl, -CO₂(C₁-C₆)alkyl, -SO₂(C₁-C₆)alkyl, -CO₂-phenyl or -CO-phenyl wherein each phenyl is unsubstituted or substituted by one or more radicals selected from the group consisting of halogen, (C₁-C₂)alkyl, (C₁-C₂)haloalkyl, (C₁-C₂)alkoxy, (C₁-C₂)haloalkoxy and NO₂);
more preferably R³ is H, (C₁-C₃)alkyl, (C₁-C₃)haloalkyl, allyl, propargyl, CHO, -CO(C₁-C₃)alkyl or -CO(C₁-C₃)haloalkyl; most preferably R³ is H, CHO, COCH₃, COCH₂Cl, COCH(CH₃)Cl or COCF₃.

Independently of the radicals R¹, R², R³, X and (Y)ₙ and preferably in combination with preferred meanings of one or more of these radicals, the following meanings of R⁴ are of particular interest.
Preferably R⁴ is H, (C₁-C₄)alkyl or (C₁-C₄)haloalkyl; more preferably R⁴ is H or (C₁-C₂)alkyl; most preferably R⁴ is H.

Independently of the radicals R¹, R², R⁴, R³ and X and preferably in combination with preferred meanings of one or more of these radicals, the following meanings of (Y)ₙ are of particular interest.
Preferably (Y)ₙ is 0, 1 or 2 radicals Y where each Y is independently (C₁-C₃)alkyl, halogen or (C₁-C₃)alkoxy; more preferably (Y)ₙ is 0, 1 or 2 radicals where each Y is independently methyl, F, Cl or methoxy (for instance in the 5 or 6 position of the indane ring).

Preferred compounds of formula (I) are those in which:
R¹ is (C₁-C₄)alkyl, such as methyl, ethyl, n-propyl or iso-propyl; or (C₁-C₃)haloalkyl, [(C₁-C₂)alkoxy](C₁-C₃)alkyl, (C₂-C₃)alkenyl, (C₂-C₃)haloalkenyl, (C₅-C₆)cycloalkenyl, (C₅-C₆)halocycloalkenyl, (C₁-C₃)alkoxy or (C₁-C₃)haloalkoxy; or is (C₃-C₅)cycloalkyl or (C₃-C₅)halocycloalkyl which cycloalkyl groups are unsubstituted or substituted by one or two methyl or ethyl groups;
R² is (C₁-C₄)alkyl, (C₁-C₃)haloalkyl, (C₃-C₅)cycloalkyl or (C₃-C₅)halocycloalkyl;
X is halogen or OH;
R³ is H, (C₁-C₄)alkyl, (C₁-C₄)haloalkyl, (C₃-C₄)alkenyl, (C₃-C₄)alkynyl or an acyl radical having 1 to 12 carbon atoms (preferably CHO, -CO(C₁-C₆)alkyl, -CO(C₁-C₆)haloalkyl, -CO₂(C₁-C₆)alkyl, -SO₂(C₁-C₆)alkyl, -CO₂-phenyl or -CO-phenyl wherein each phenyl is unsubstituted or substituted by one or more radicals selected from the group consisting of halogen, (C₁-C₂)alkyl, (C₁-C₂)haloalkyl, (C₁-C₂)alkoxy, (C₁-C₂)haloalkoxy and NO₂);
R⁴ is H, (C₁-C₄)alkyl or (C₁-C₄)haloalkyl; and
(Y)ₙ is 0, 1 or 2 radicals Y where each Y is independently (C₁-C₃)alkyl, halogen or (C₁-C₃)alkoxy.

More preferred compounds of formula (I) are those in which:
R¹ is (C₁-C₃)alkyl;
R² is (C₁-C₄)alkyl or (C₃-C₅)cycloalkyl;
X is Cl, F or OH;
R³ is H, (C₁-C₃)alkyl, (C₁-C₃)haloalkyl, allyl, propargyl, CHO, -CO(C₁-C₃)alkyl or -CO(C₁-C₃)haloalkyl;
R⁴ is H or (C₁-C₂)alkyl; and
(Y)ₙ is 0, 1 or 2 radicals where each Y is independently (C₁-C₃)alkyl, halogen or (C₁-C₃)alkoxy.

Especially preferred compounds of formula (I) are those in which:
R¹ is (C₁-C₂)alkyl;
R² is (C₁-C₃)alkyl or (C₃-C₅)cycloalkyl;
X is Cl or F;
R³ is H, CHO, COCH₃, COCH₂Cl, COCH(CH₃)Cl or COCF₃;
R⁴ is H; and
(Y)ₙ is 0, 1 or 2 radicals where each Y is independently methyl, F, Cl or methoxy.

A preferred embodiment of the invention relates to optically active compounds of formula (la): 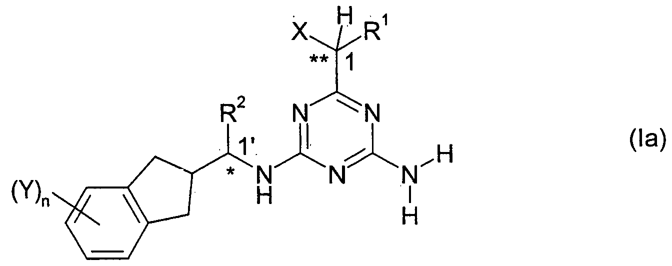 in which the various symbols are as defined in formula (I) and salts thereof, where the optically active compound in question is present as a pure stereoisomer (having *R* or *S* configuration) with respect to the configuration of the carbon atom marked in formula (la) with a double asterisk (**), or the carbon atom marked in formula (la) with an asterisk (*), or as a mixture of the *R* and *S* stereoisomers with an excess of one of the stereoisomers, preferably a content of *R* or *S* isomer of from 60 to 100%, in particular from 70 to 100%, especially from 80 to 100%, most preferably from 90 to 100%, based on the total amount of *R* and *S* isomers.

The following stereoisomers of the compounds of general formula (la) wherein the configurations at the carbon atoms marked with (**) and (*) are specifically defined as formula (Ib), (Ic), (Id) or (le) are also preferred:

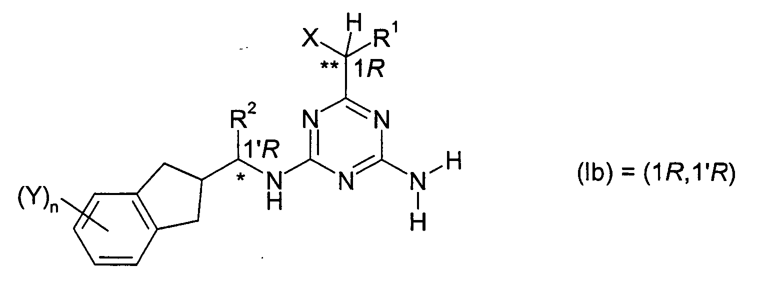

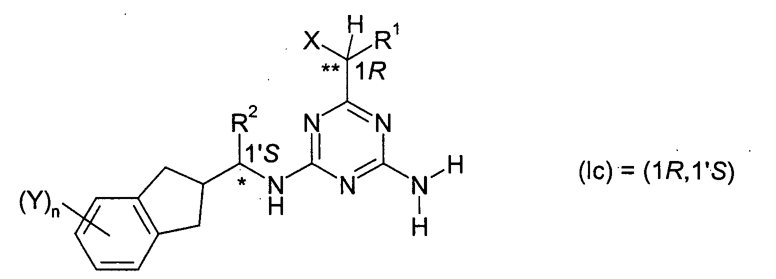

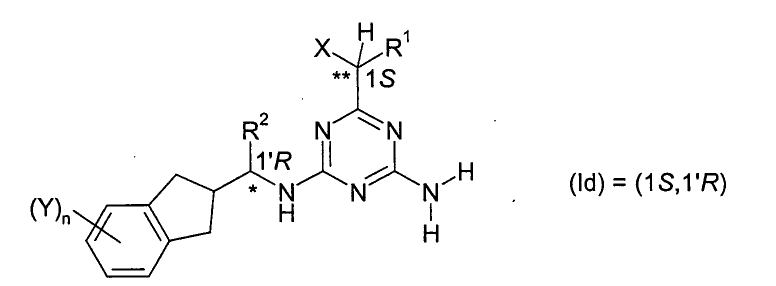

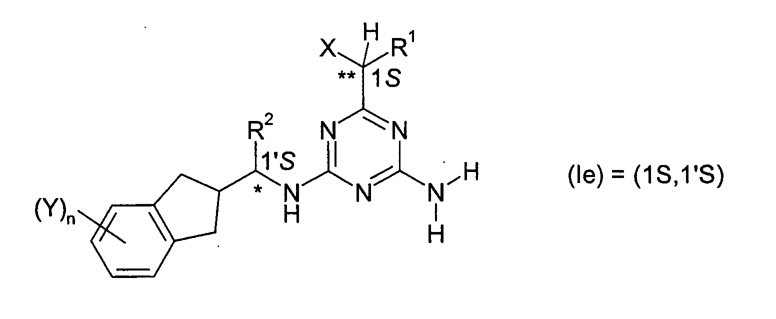

A further class of preferred compounds are of formula (la), (Ib), (Ic), (Id) or (le) in which the radicals R¹, R², X, Y and n are as defined in the preferred meanings for formula (I).

A further class of especially preferred compounds are of formula (la), (Ib), (Ic), (Id) or (le) in which:
R¹ is (C₁-C₄)alkyl;
R² is (C₁-C₃)alkyl or (C₃-C₅)cycloalkyl;
X is Cl or F;
R³ is H, CHO, COCH₃, COCH₂Cl, COCH(CH₃)Cl or COCF₃;
R⁴ is H; and
(Y)ₙ is 0, 1 or 2 radicals where each Y is independently methyl, F, Cl or methoxy.

Compounds of formula (I) above may be prepared by the application or adaptation of known methods (i.e. methods heretofore used or described in the literature), for example as generally described in WO 97/29095 and references cited therein, and as hereinafter described.
In the following description where symbols appearing in formulae are not specifically defined, it is to be understood that they are "as hereinbefore defined" in accordance with the first definition of each symbol in the specification.
It is to be understood that in the descriptions of the following processes the sequences may be performed in different orders, and that suitable protecting groups may be required to achieve the compounds sought.

According to a feature of the present invention compounds of formula (I) wherein R¹, R², R³, R⁴, X, Y and n are as defined above, may be prepared by the reaction of a compound of general formula (II): 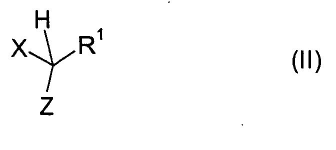 wherein R¹ and X are as defined in formula (I), and Z is a functional group selected from the group consisting of carboxylic ester, carboxylic orthoester, carboxylic acid chloride, carboxamide, cyano, carboxylic anhydride or trichloromethyl, with a biguanidine compound of formula (III) or an acid addition salt thereof: 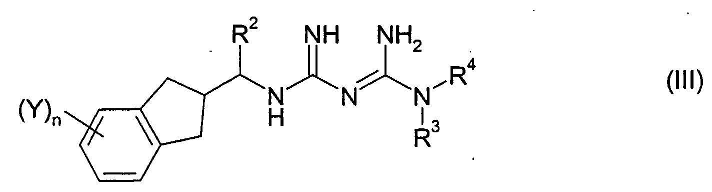 wherein R², R³, R⁴, Y and n are as defined in formula (I). The reaction is generally performed in the presence of a base, in an inert solvent such as tetrahydrofuran, dioxan, acetonitrile, N,N-dimethylformamide, methanol or ethanol, at a temperature of from 0°C to the reflux temperature of the solvent, preferably at 20°C to 60°C. The base is generally an alkali metal hydroxide, alkali metal hydride, alkali metal carbonate, alkali metal alkoxide, alkaline earth metal carbonate, or an organic base such as,a tertiary amine for example triethylamine, or 1,8-diazabicyclo[5.4.0]undec-7-en (DBU).

According to a further feature of the present invention compounds of formula (I) wherein R¹, R², R³, R⁴, X, Y and n are as defined above, may be prepared by the reaction of a compound of general formula (IV): 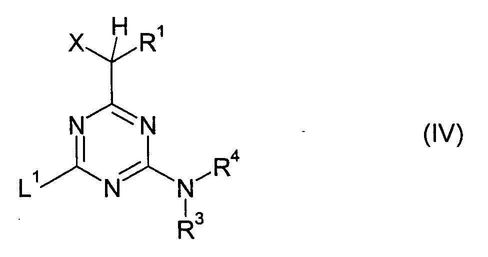 wherein R¹, R³, R⁴ and X are as defined in formula (I), L¹ is a leaving group such as chlorine, trichloromethyl, (C₁-C₄)-alkylsulfonyl, phenylsulfonyl or (C₁-C₄)alkylphenylsulfonyl, with an amine of formula (V) or an acid addition salt thereof: 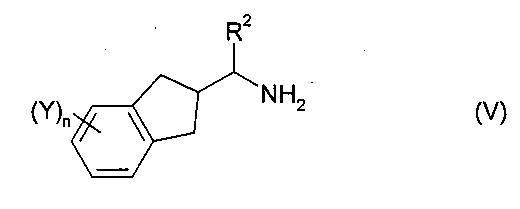 wherein R², Y and n are as defined in formula (I). The reaction is generally performed in the presence of a base, in an inert solvent such as tetrahydrofuran, dioxan, acetonitrile, N,N-dimethylformamide, methanol or ethanol, at a temperature of from 0°C to the reflux temperature of the solvent, preferably at 20°C to 100°C. The base is generally an alkali metal hydroxide, alkali metal hydride, alkali metal carbonate, alkali metal alkoxide, alkaline earth metal carbonate, or an organic base such as a tertiary amine for example triethylamine, or 1,8-diazabicyclo[5.4.0]undec-7-en (DBU).
The process is known in general terms from for example Comprehensive Heterocyclic Chemistry, A.R. Katritzky, C.W. Rees, Pergamon Press, Oxford, New York, 1984, Vol.3; Part 2B; ISBN 0-08-030703-5, S. 482.

According to a further feature of the present invention compounds of formula (I) wherein R¹, R², X, Y and n are as defined above, and one of R³ or R⁴ is (C₁-C₄)alkyl or (C₁-C₄)haloalkyl, and the other of R⁴ and R³ respectively is as defined above, may be prepared by the alkylation of the corresponding compound of formula (I) wherein R³ or R⁴ respectively is H, with an alkylating agent of formula (VI) or (VII) respectively:

R³-L² (VI)

R⁴-L² (VII)

wherein R³ and R⁴ are each (C₁-C₄)alkyl, (C₁-C₄)haloalkyl, and L² is a leaving group, generally halogen, preferably chlorine, bromine or iodine, or an alkyl- or phenylsulfonyloxy moiety such as methyl sulfonyloxy or 4-toluenesulfonyloxy. The reaction is generally performed in an inert solvent such as tetrahydrofuran, dioxan, acetonitrile or N,N-dimethylformamide at a temperature of from 0°C to the reflux temperature of the solvent, preferably at 20°C to 100°C.

According to a further feature of the present invention compounds of formula (I) wherein R¹, R², X, Y and n are as defined above, one of R³ or R⁴ is an acyl radical, and the other of R⁴ or R³ respectively is as defined above, may be prepared by the reaction of the corresponding compound of formula (I) wherein R³ or R⁴ respectively is H, with an acylating agent of formula (VIII) or (IX) respectively:

R³-L³ (VIII)

R⁴-L³ (IX)

wherein R³ and R⁴ are each an acyl radical as defined above and L³ is a leaving group, generally halogen preferably chlorine; or with a formylating agent such as formic acid-acetic anhydride. A base is optionally used for the acylation reaction and is generally chosen from an alkali metal hydroxide, alkali metal hydride, alkali metal carbonate, alkali metal alkoxide, alkaline earth metal carbonate, or an organic base such as a tertiary amine for example triethylamine.
The reaction is generally performed in an inert solvent such as tetrahydrofuran, dioxan, acetonitrile or N,N-dimethylformamide at a temperature of from 0°C to the reflux temperature of the solvent, preferably at 20°C to 100°C.

Intermediates of formula (III) may be prepared by the reaction of a compound of formula (X): 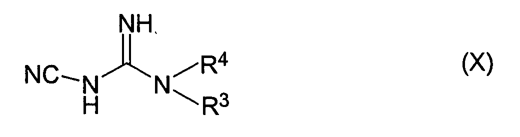 with a compound of formula (V). The reaction is generally performed using an acid addition salt for example the hydrochloride salt of the compound of formula (X), in a solvent such as 1,2-dichlorobenzene, at a temperature of from 20°C to the reflux temperature of the solvent, preferably at 50°C to 200°C.

Intermediates of formula (V) may be prepared according to known methods, for example by the reductive amination of ketones of formula (XI): 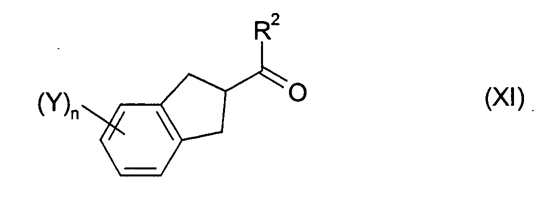 in which R² and (Y)ₙ are as defined above, or the reaction of compounds of formula (XII): 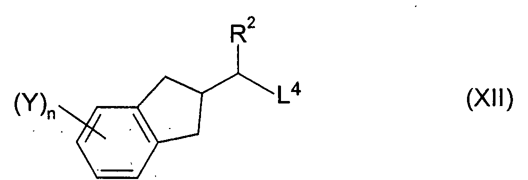 in which R² and (Y)ₙ are as defined above and L⁴ is a leaving group such as halogen, hydroxy, methyl sulfonyloxy or 4-toluenesulfonyloxy, with ammonia or a salt thereof, according to known procedures for example as described in WO 97/00254, WO 97/08156 or WO 99/37627. Ketones of formula (XI) may be prepared according to methods, for example as described in Synthesis 1995, 139.

For the preparation of compounds of formula (I), in which one or more asymmetric carbon atoms is present as a single enantiomeric form, the above processes can be adapted by employing the appropriate enantiomeric or diastereomeric form of the compounds of formula (II), (III), (IV) or (V).
Compounds of formula (II) in an enantiomerically pure form are known or can be performed according to known procedures, for example as described in Tetrahedron Asymmetry 1994, 5, 981, J. Chem. Soc. Perkin Trans 1,1979, 2248 and the literature cited therein.

It is generally possible to use customary methods for optical resolutions (cf. Textbooks of Stereochemistry), for example following processes for separating mixtures into diastereomers, for example physical processes, such as crystallization, chromatographic processes, in particular column chromatography and high pressure liquid chromatography, distillation, if appropriate under reduced pressure, extraction and other processes, it is possible to separate the remaining mixtures of enantiomers, generally by chromatographic separation on chiral solid phases. Suitable for preparative amounts or use on an industrial scale are processes such as the crystallization of diastereomeric salts which can be obtained from the compounds (I) using optically active acids and, if appropriate, provided that acidic groups are present, using optically active bases.

Optically active acids which are suitable for optical resolution by crystallization of diastereomeric salts are, for example, camphorsulfonic acid, camphoric acid, bromocamphorsulfonic acid, quinic acid, tartaric acid; dibenzoyltartaric acid and other analogous acids; suitable optically active bases are, for example, quinine, cinchonine, quinidine, brucine, 1-phenylethylamine and other analogous bases.

The crystallizations are then in most cases carried out in aqueous or aqueous-organic solvents, where the diastereomer which is less soluble precipitates first, if appropriate after seeding. One enantiomer of the compound of the formula (I) is then liberated from the precipitated salt, or the other is liberated from the crystals, by acidification or using base.

Enantiomerically pure amine intermediates of formula (V) may be prepared using known methods, for example as described in Houben-Weyl, Methoden der Organischen Chemie, 4. Auflage, Band E 21b, 1833 ff. or Band E 21e, 5133 ff.).
One of the preferred procedures is the reductive amination of ketones of formula (XI) with asymmetric catalysis. Another procedure for the preparation of enantiomerically pure amines of formula (V), is the racemate cleaving method described in *J. Prakt. Chem.* 339, (1997). In this procedure the racemic amine of the general formula (V) is acylated enantioselectively with an optionally substituted fatty acid ester (preferably methyl- or ethyl- chloroacetate or methyl- or ethylmethoxyacetate) in the presence of a biocatalyst. The non acylated enantiomer is then separated by simple treatment with mineral acid. The acylated amine enantiomer is then cleaved back to the corresponding amine, generally using a base for example an alkali metal hydroxide such as sodium hydroxide.
As biocatalysts, lipases, for example *pseudomonas cepacia, candida cylindracea or candida antarctica,* are particularly suitable for this purpose. Certain of these lipases are also commercially available in immobilized form (brand name: "Novozym 435").

The following acids, for example, are suitable for preparing the acid addition salts of the compounds of the formula (I): hydrohalic acids, such as hydrochloric acid or hydrobromic acid, furthermore phosphoric acid, nitric acid, sulfuric acid, mono- or bifunctional carboxylic acids and hydroxycarboxylic acids, such as acetic acid, oxalic acid, maleic acid, succinic acid, fumaric acid, tartaric acid, citric acid, salicylic acid, sorbic acid or lactic acid, and also sulfonic acids, such as p-toluenesulfonic acid and 1,5-naphthalenedisulfonic acid. The acid addition compounds of the formula (I) can be obtained in a simple manner by the customary methods for forming salts, for example by dissolving a compound of the formula (I) in a suitable organic solvent, such as, for example, methanol, acetone, methylene chloride or benzene, and adding the acid at temperatures from 0 to 100°C, and they can be isolated in a known manner, for example by filtration, and, if appropriate, purified by washing with an inert organic solvent.

The base addition salts of the compounds of the formula (I) are preferably prepared in inert polar solvents such as, for example, water, methanol or acetone at temperature from 0 to 100°C. Examples of suitable bases for preparing the salts according to the invention are alkali metal carbonates, such as potassium carbonate, alkali metal and alkaline earth metal hydroxides, for example NaOH or KOH, alkali metal and alkaline earth metal hydrides, for example NaH, alkali metal and alkaline earth metal alkoxides, for example sodium methoxide, potassium tert-butoxide, or ammonia or ethanolamine. Quaternary ammonium salts can be obtained, for example, by salt exchange or condensation with quaternary ammonium salts of the formula [NRR'R''R''']⁺X⁻ where R, R', R" and R''' independently of one another are (C₁-C₄)alkyl, phenyl or benzyl and X' is an anion, for example Cl⁻ or OH⁻.

Some of the compounds of formula (III) and (V) in which one or more asymmetric carbon atoms are present as a single or optically enriched enantiomeric form are novel and as such form a further feature of the invention, and may be prepared as described above.
Compounds of formula (II), (IV), (VI), (VII), (VIII), (IX), (X), (XI) and (XII), as well as racemic (III) and (V) are known or may be prepared by according to known methods. For example compounds of formula (V) may be prepared from 1,2-(bis-halomethyl)benzenes by reaction with acetylacetone in the presence of a base, to give the corresponding 2-acetylindane, followed by reductive amination using a source of ammonia, for example ammmonium acetate, and a reducing agent such as sodium cyanoborohydride, sodium borohydride or hydrogen and a catalyst.

A collection of compounds of formula (I) which can be synthesized by the abovementioned processes can additionally be prepared in parallel fashion, which can be effected manually, partly automated or fully automated. In this context, it is possible to automate the procedure of the reaction, work-up or purification of the products or intermediates. In total, this is to be understood as meaning a procedure which is described, for example, by S. H. DeWitt in "Annual Reports in Combinatorial Chemistry and Molecular Diversity: Automated Synthesis", Volume 1, published by Escom, 1997, pages 69 to 77.

For carrying out the reaction and work-up in parallel fashion, a series of commercially available apparatuses can be used as they are available from, for example, Stem Corporation, Woodrolfe Road, Tollesbury, Essex, CM9 8SE, England or H + P Labortechnik GmbH, Bruckmannring 28, 85764 Oberschleissheim, Germany. To carry out the parallel purification of compounds (I) or of intermediates obtained during the preparation, there are available, inter alia, chromatographic equipment, for example from ISCO, Inc., 4700 Superior Street, Lincoln, NE 68504, USA. The equipment mentioned makes possible a modular procedure, where the individual steps are automated, but manual operation has to be carried out between the steps. This can be circumvented by employing partly or fully integrated automation systems, in which the automation modules in question are operated by, for example, robots. Such automation systems can be obtained from, for example, Zymark Corporation, Zymark Center, Hopkinton, MA 01748, USA.

In addition to the above-described methods, compounds of formula (I) can be prepared in full or partly by solid-phase supported methods. To this end, individual intermediates or all intermediates of the synthesis or of a synthesis adapted to the procedure in question are bound to a synthesis resin. Solid-phase supported synthetic methods are described extensively in the specialist literature, for example: Barry A. Bunin in "The Combinatorial Index", published by Academic Press, 1998. The use of solid-phase supported synthesis methods permits a series of protocols known from the literature which, in turn, can be carried out manually or in an automated fashion. For example, the "teabag method" (Houghten, US 4,631,211; Houghten et al., Proc. Natl. Acad. Sci., 1985, 82, 5131 - 5135) can be partly automated with products of IRORI, 11149 North Torrey Pines Road, La Jolla, CA 92037, USA. Solid-phase supported parallel synthesis can be automated successfully for example using equipment by Argonaut Technologies, Inc., 887 Industrial Road, San Carlos, CA 94070, USA or MultiSynTech GmbH, Wullener Feld 4, 58454 Witten, Germany.

The preparation in accordance with the processes described herein yields compounds of formula (I) in the form of substance collections or substance libraries. Subject matter of the present invention are therefore also libraries of the compounds of formula (I) which contain at least two compounds of formula (I), and of their precursors.

The following non-limiting Examples illustrate the preparation of the compounds of formula (I).

### A. Chemical Examples

In the Examples which follow, quantities (also percentages) are weight based unless stated otherwise. Ratios of solvents are volume based.

### Example A1

### rac-2-Amino-4-(1-fluoropropyl)-6-[1-(indan-2-yl)-1-propylamino]-1,3,5-triazine (Compound No. 1.36 in Table 1)

A mixture of 1-amino-1-(indan-2-yl)propane hydrochloride (1.00 g, 5.7 mmol) and finely powdered 1-cyanoguanidine (0.48 g, 5.7 mmol) was heated in 1,2-dichlorobenzene for 3 hours at 160°C. The cooled mixture was filtered, washed with heptane and dried to give 1-bisguanidino-1-(indan-2-yl)propane hydrochloride (1.35 g, 91% of theory) as colourless crystals.
To a solution of 1.05 g (4.05 mmol) of the above obtained 1-bisguanidino-1-(indan-2-yl)propane hydrochloride in methanol was added a solution of sodium (195 mg) in methanol. Racemic ethyl 2-fluorobutyrate (1.00 g, 7.5 mmol) was then added and the mixture stirred for 3 hours at 20°C, then for 4 hours at 65°C. The solvent was evaporated and the residue dissolved in dichloromethane, washed (water), dried (sodium sulfate) and evaporated to give *rac*-2-amino-4-(1-fluoropropyl)-6-[1-(indan-2-yl)-1-propylamino]-1,3,5-triazine (Compound No. 1.36, 1.20 g, 91% of theory).

By proceeding in a similar manner but using *rac*-1-bisguanidino-1-(indan-2-yl)ethane hydrochloride (1.20 g, 4.3 mmol) and methyl (*S*)-2-fluoropropionate (0.90 g, 8.5 mmol) there was obtained a mixture of 2-amino-4-(1-(*S*)-fluoroethyl)-6-[1-(indan-2-yl)-1-(*S*)-ethylamino]-1,3,5-triazine and 2-amino-4-(1-(*S*)-fluoroethyl)-6-[1-(indan-2-yl)-1-(*R*)-ethylamino]-1,3,5-triazine (Compound No. 1.66, 1.11 g, 86% of theory) as colourless crystals, which may be analysed by chiral HPLC.

### Example A2

### rac-2-Amino-4-(1-fluoroethyl)-6-[1-(indan-2-yl)-1-propylamino]-1,3,5-triazine (Compound No. 1.105 in Table 1)

A mixture of 1-amino-1-(indan-2-yl)-propane (0.55 g, 3.1 mmol), 2-amino-4-chloro-6-(1-fluoroethyl)-1,3,5-triazine (0.55 g, 3.1 mmol) and potassium carbonate (1.00 g, 7.2 mmol) was heated under reflux in acetonitrile for 2 hours. The cooled mixture was filtered, evaporated and purified by chromatography on Kieselgel (eluting with ethyl acetate-hexane, 1:1) to give *rac*-2-amino-4-(1-fluoroethyl)-6-[1-(indan-2-yl)-1-propylamino]-1,3,5-triazine (Compound No. 1.105, 0.86 g, 87% of theory).

### Example A3

### rac-2-Acetylamino-4-(1-chloroethyl)-6-[1-(indan-2-yl)-1-ethylamino]-1,3,5-triazine (Compound No. 2.1, Table 2)

A mixture of 2-amino-4-(1-chloroethyl)-6-[1-(indan-2-yl)-1-ethylamino]-1,3,5-triazine (Compound No. 1.121 in Table 1, 0.20 g, 0.63 mmol) and acetyl chloride (0.1 g) in toluene was heated under reflux for 2 hours, evaporated and purified by chromatography on Kieselgel (eluting with ethyl acetate-hexane, 1:3) to give *rac*-2-acetylamino-4-(1-chloroethyl)-6-[1-(indan-2-yl)-1-ethylamino]-1,3,5-triazine (Compound No. 2.1, 0.21 g, 92% of theory) as colourless crystals.

The following preferred compounds of formula (I) shown in Tables 1 and 2 also form part of the present invention, and are obtained by, or analogously to, the above Examples A1, A2 and A3 or the above-described general methods.

The following abbreviations are used in the Tables 1, 2 and 3:
Me means methyl, Et means ethyl, Pr means n-propyl, iPr means iso-propyl, cPr means cyclopropyl, cBu means cyclobutyl, cPe means cyclopentyl and OMe means methoxy.
"H" for Y means no substitution by (Y)ₙ (n = 0).
Cpd means Compound Number. Compound numbers are given for reference purposes only.
¹H-NMR Spectra were recorded in deuterochloroform unless otherwise stated. The following notations are used: s = singlet, d = doublet, t = triplet, m = multiplet, mc = centred mutiplet.
The following HPLC methods were used:
HPLC-Method 1: Chiracel OD (Daicel, 250x4.6 mm); n-hexane:2-propanol v/v 85:15, flow 1 ml/min; detection UV 210 nm;
HPLC-Method 2: Chiracel OD (Daicel 250x4.6 mm); n-hexane:2-propanol v/v 90:10, flow 0.6 ml/min; Detection UV 210 nm;
HPLC-Method 3: Chiracel OD (Daicel, 250x4.6 mm); n-hexane:2-propanol v/v 95: 5, flow 1ml/min; Detection UV 210 nm;
HPLC-Method 4: YMC ODS-AM 5µm (250x4.6 mm); eluting with acetonitrile and a mixture of 0.05% trifluoroacetic acid in water, linear gradient v/v 1:99 to 80:20 within 30 minutes, flow 1ml/min; detection UV 210 nm.
mins means minutes.
Abbreviations for the notation of stereoisomers are used according to the Cahn-Ingold-Prelog system (R,S notation) in the usual manner.
The following are examples of abbreviations used:for the notation of stereoisomers of compounds of formula (If):
(1*R*,1'*R*,2"*R*) means a compound of formula (If) having the *R* configuration at each chiral centre;
(1*R*,1'*S*,2"*R*) means a compound of formula (If) having the *R* configuration at the 1 and 2" carbon atoms and *S* configuration at the 1' carbon atom;
(1*RS*,1'*R*,2"*R*) means a diastereomeric mixture of about 1:1 of the (1*R*,1'*R*,2"*R*) and
(1*S*,1'*R*,2"*R*) compounds, being racemic at the 1 carbon atom and having the *R* configuration at the 1' and 2" carbon atoms;
racemate means herewith more generally a mixture of about equal amounts of four racemic diastereoisomers i.e. racemic at each chiral centre.

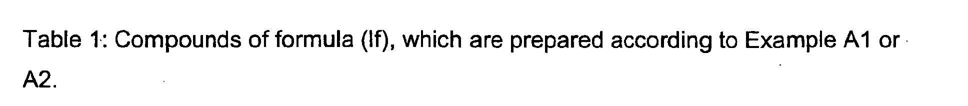

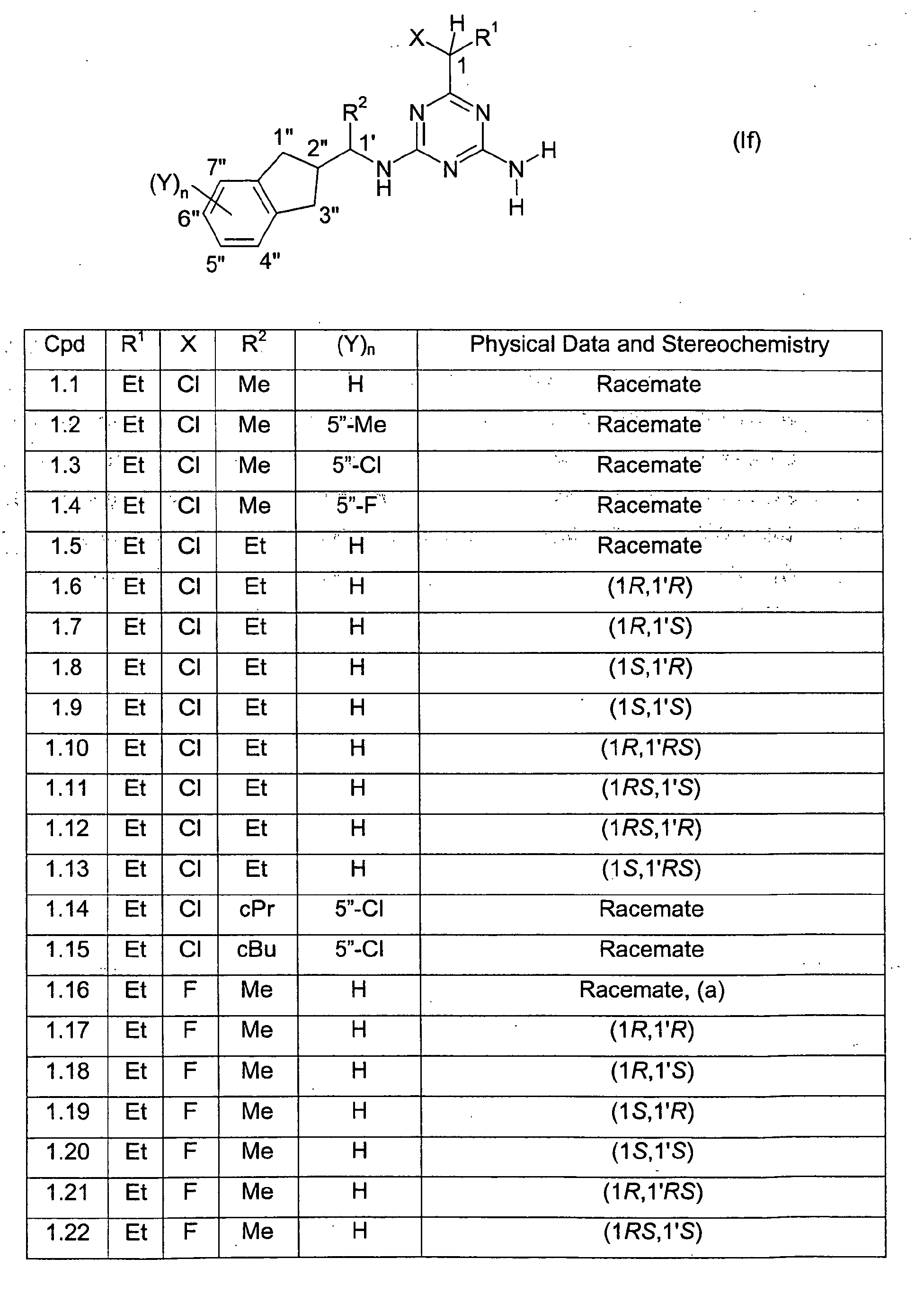

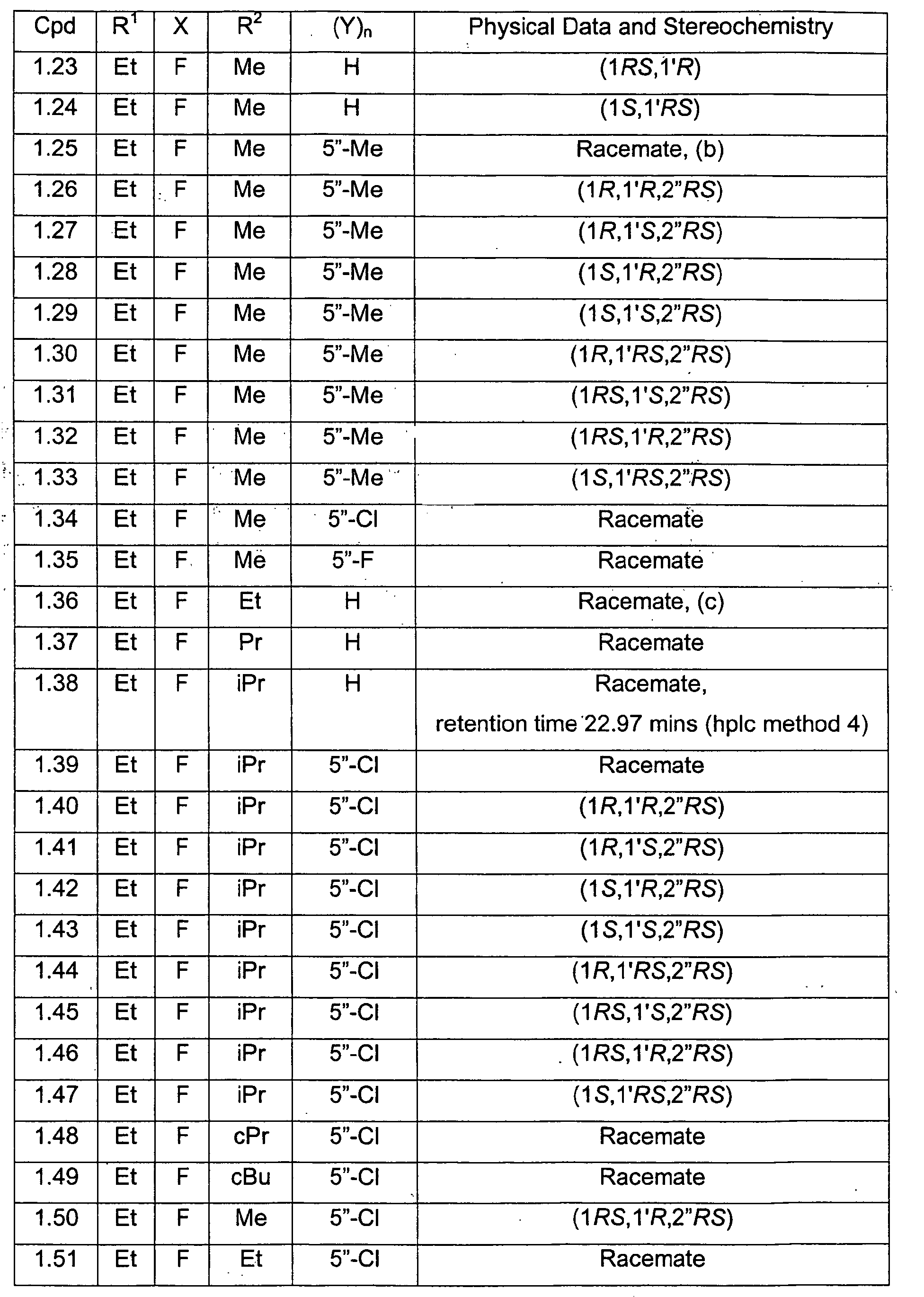

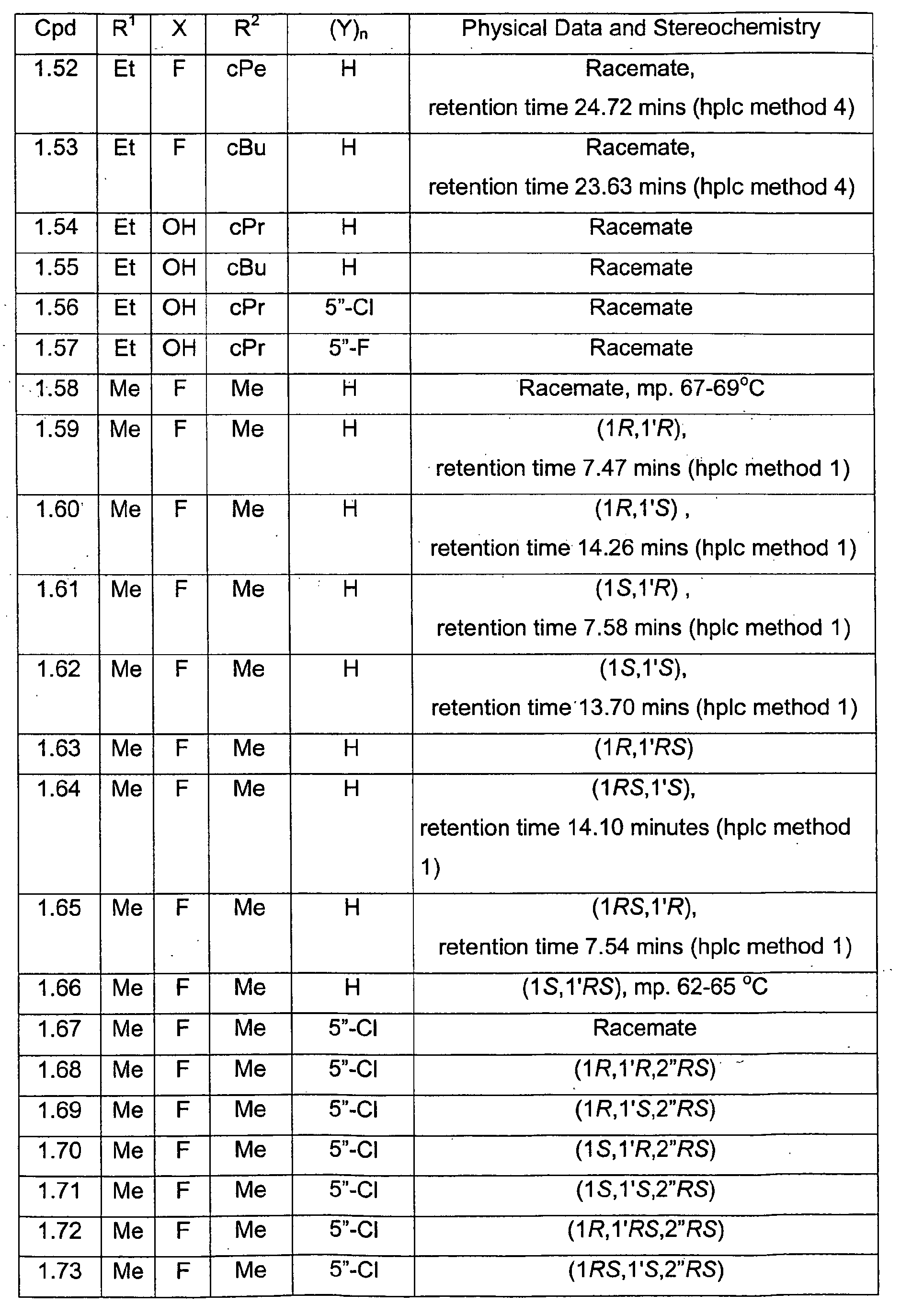

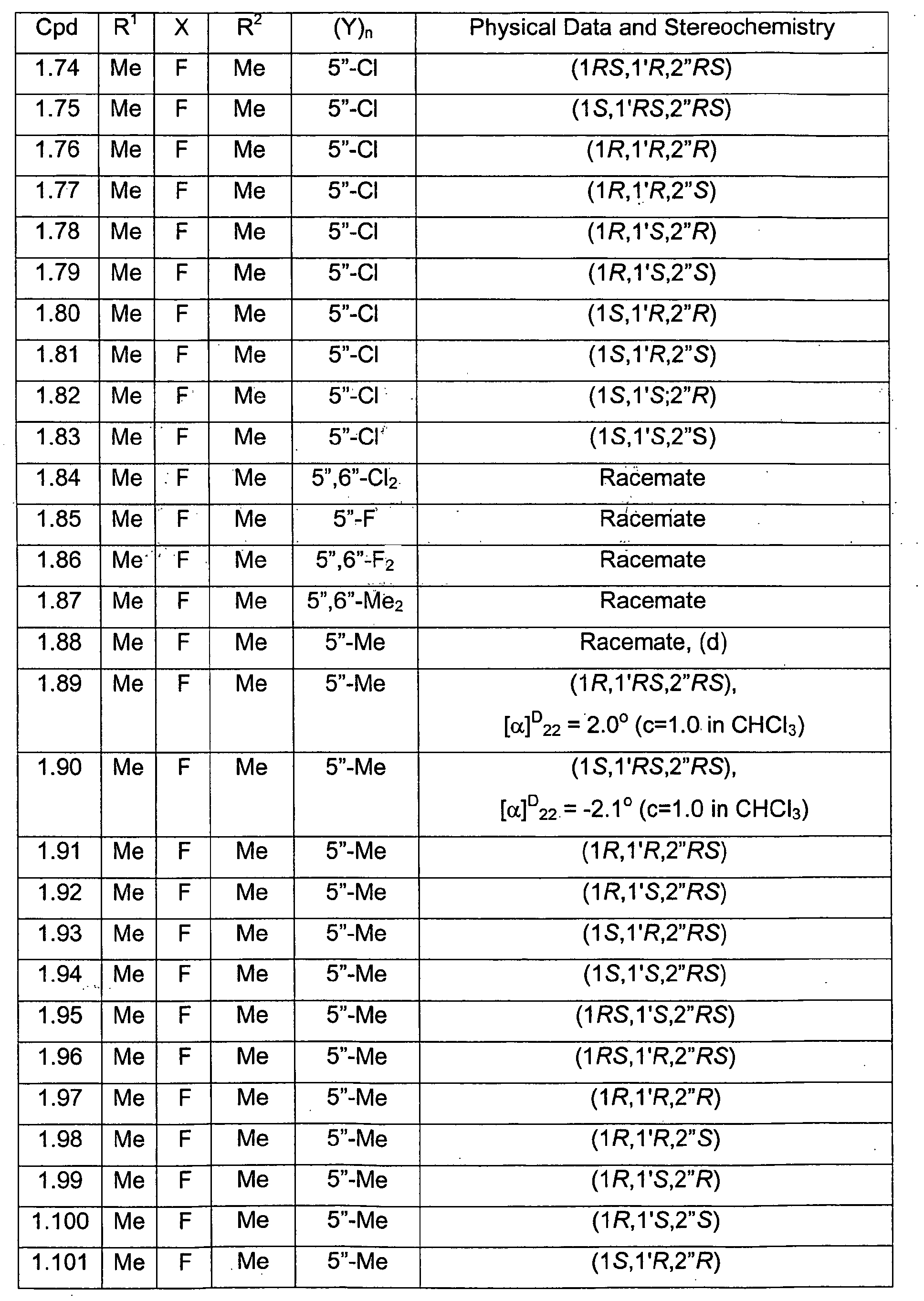

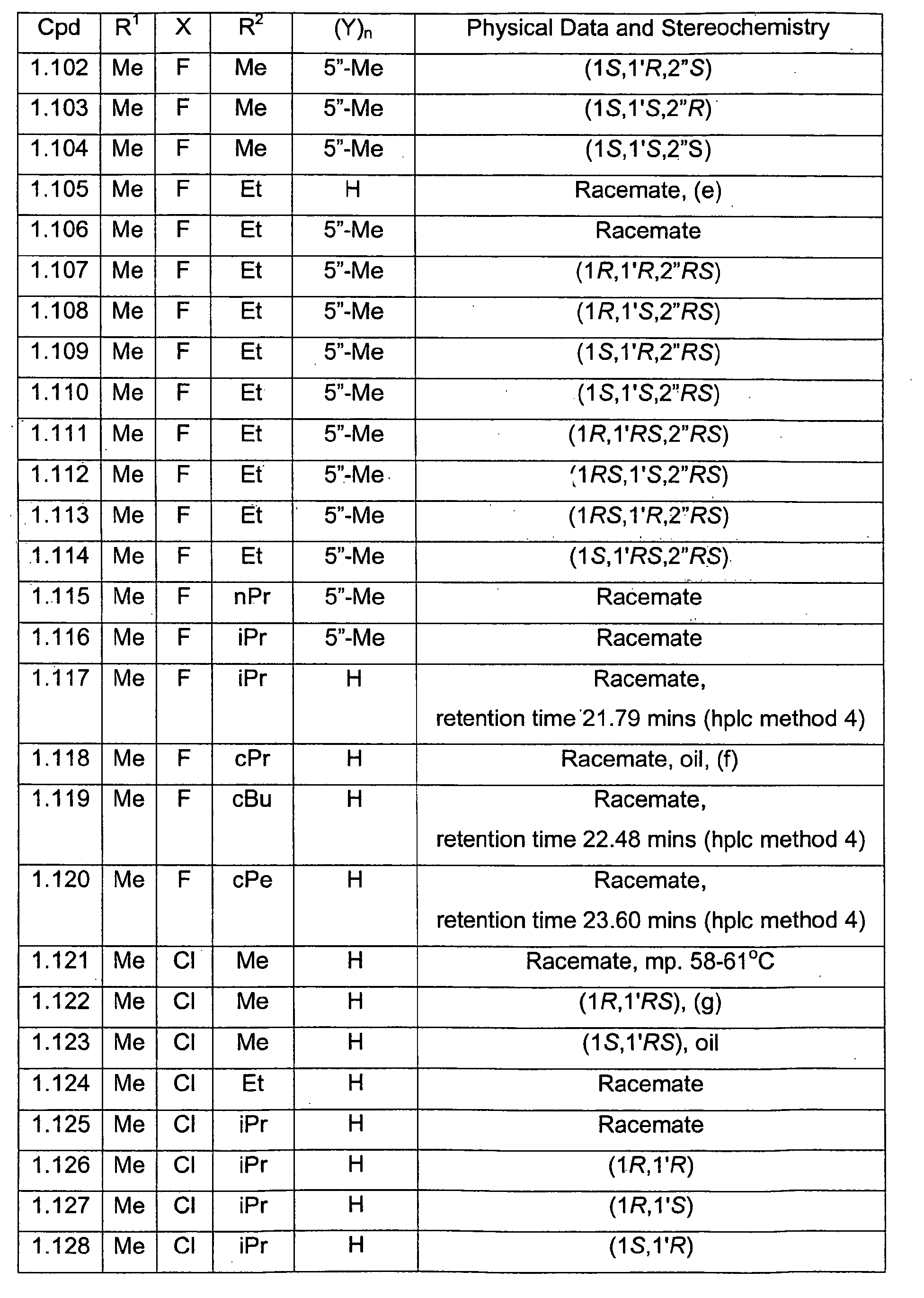

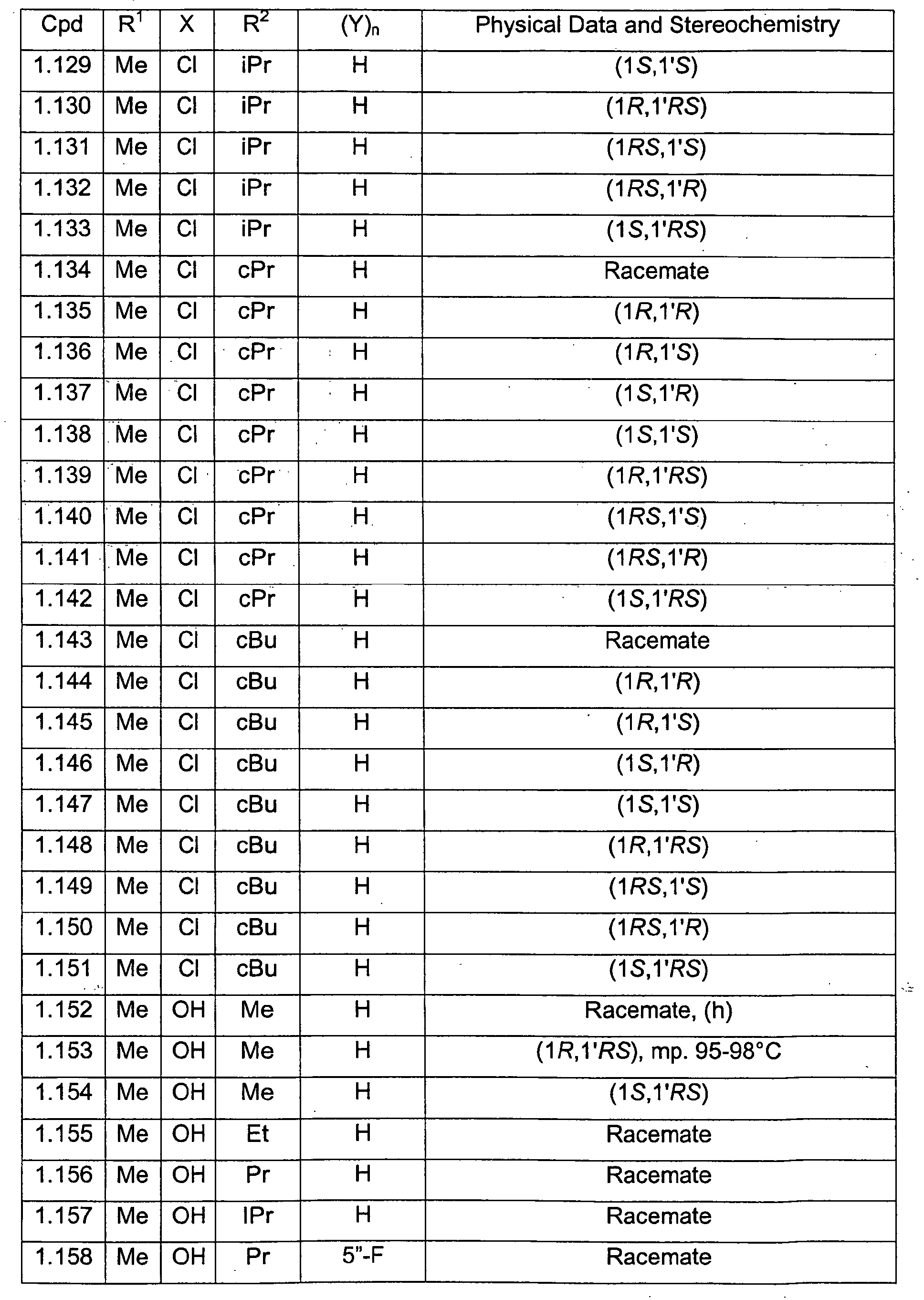

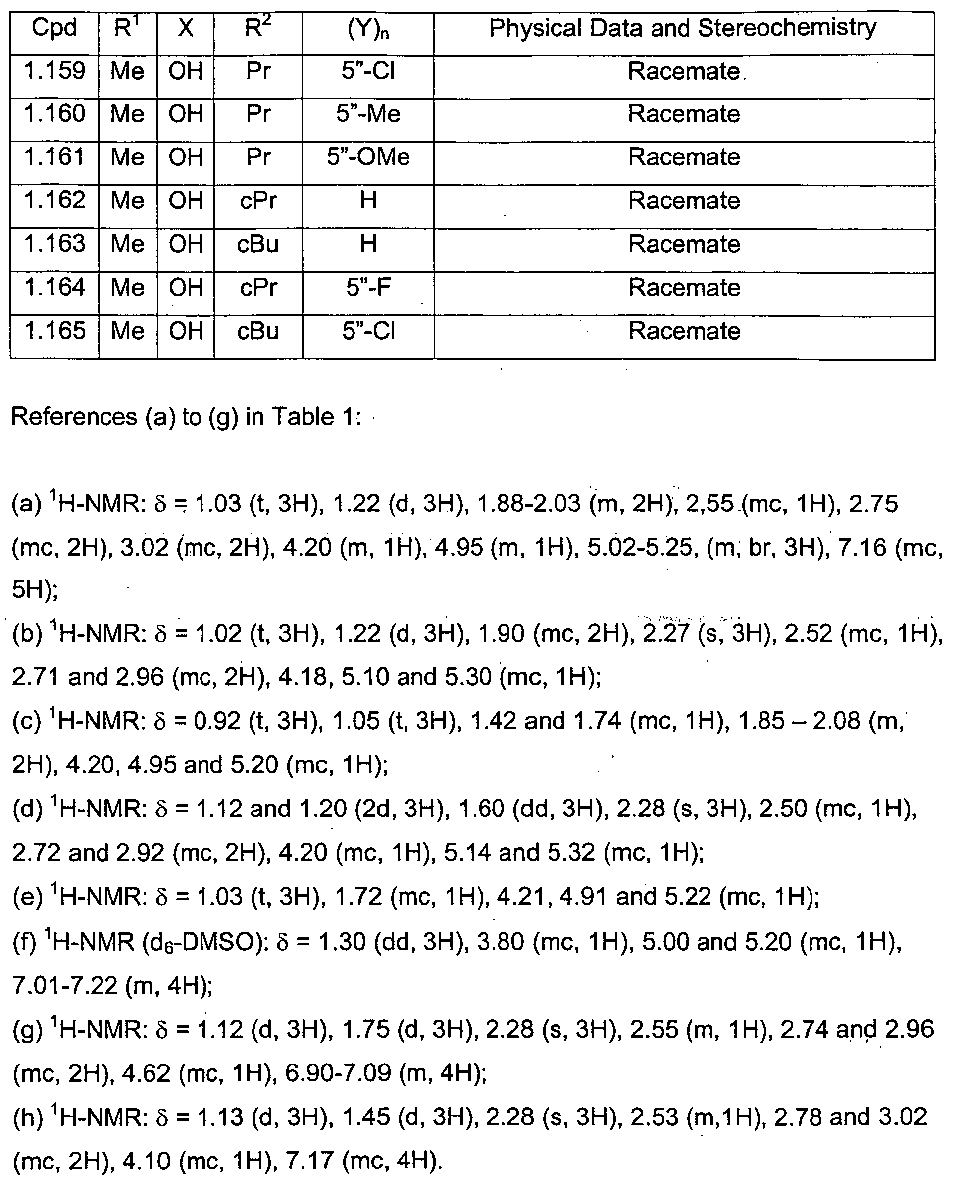

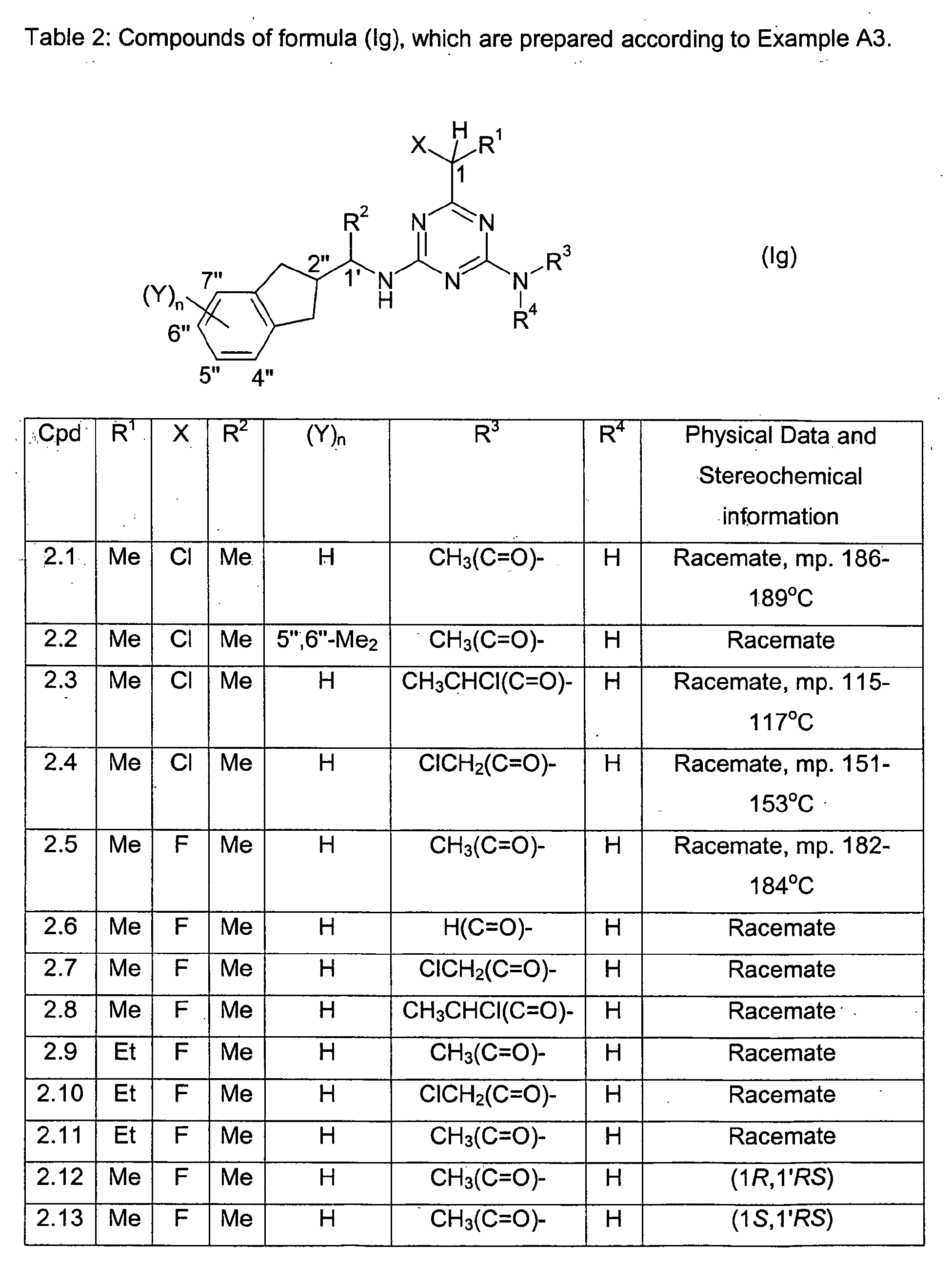

The following Example A.a illustrates the preparation of intermediates used in the synthesis of the above Examples.

### Example A.a

### 1-Amino-1-(5-methylindan-2-yl)ethane (Compound Nr. 3.21 in Table 3)

A solution of phosphoryl tribromide (2.90 g, 19 mmol) in diethylether was added dropwise to a solution of 1,2-bis-(hydroxymethyl)-4-methylbenzene (3.61 g, 13 mmol) in diethyl ether maintaining at -15°C. The mixture was stirred for 5 hours at 20°C, then washed with water, dried (magnesium sulfate) and evaporated to give 1,2-bis-(bromomethyl)-4-methylbenzene (4.39 g, 83% of theory) as a yellow solid, mp. 53-54°C.

A mixture of tetrabutylammonium bromide (4.30 g, 15.4 mmol) and the above-mentioned reaction product (3.60 g. 89 mmol) was added to a solution of sodium hydroxide (0.26 g, 0.8 mmol) in water and stirred for 10 minutes. A solution of acetylacetone (1.60 g) in toluene was added and the mixture heated at reflux for 5 hours. After cooling the organic phase was washed (water), dried (magnesium sulfate), evaporated and purified by chromatography on Kieselgel to give 2-acetyl-5-methylindane (2.32 g, 86% of theory) as colourless crystals, mp. 34-35°C.
A mixture of this 2-acetyl-5-methylindane (1.50 g, 8.6 mmol), ammonium acetate (6.63 g, 86 mmol) and sodium cyanoborohydride (0.57 g, 8.6 mmol) in methanol was stirred for 12 hours at 20°C. After concentration the residue was dissolved in dichloromethane, extracted with hydrochloric acid (2N) and the extract basified to pH 10 with sodium hydroxide solution (2N) and extracted with dichloromethane. The organic extract was dried (magnesium sulfate) and evaporated to give 1-amino-1-(5-methylindan-2-yl)ethane (1.25 g, 84 % of theory) as colourless crystals.

The amine intermediates of formula (V) set forth in Table 3 below are also prepared anagously to Example A.a:

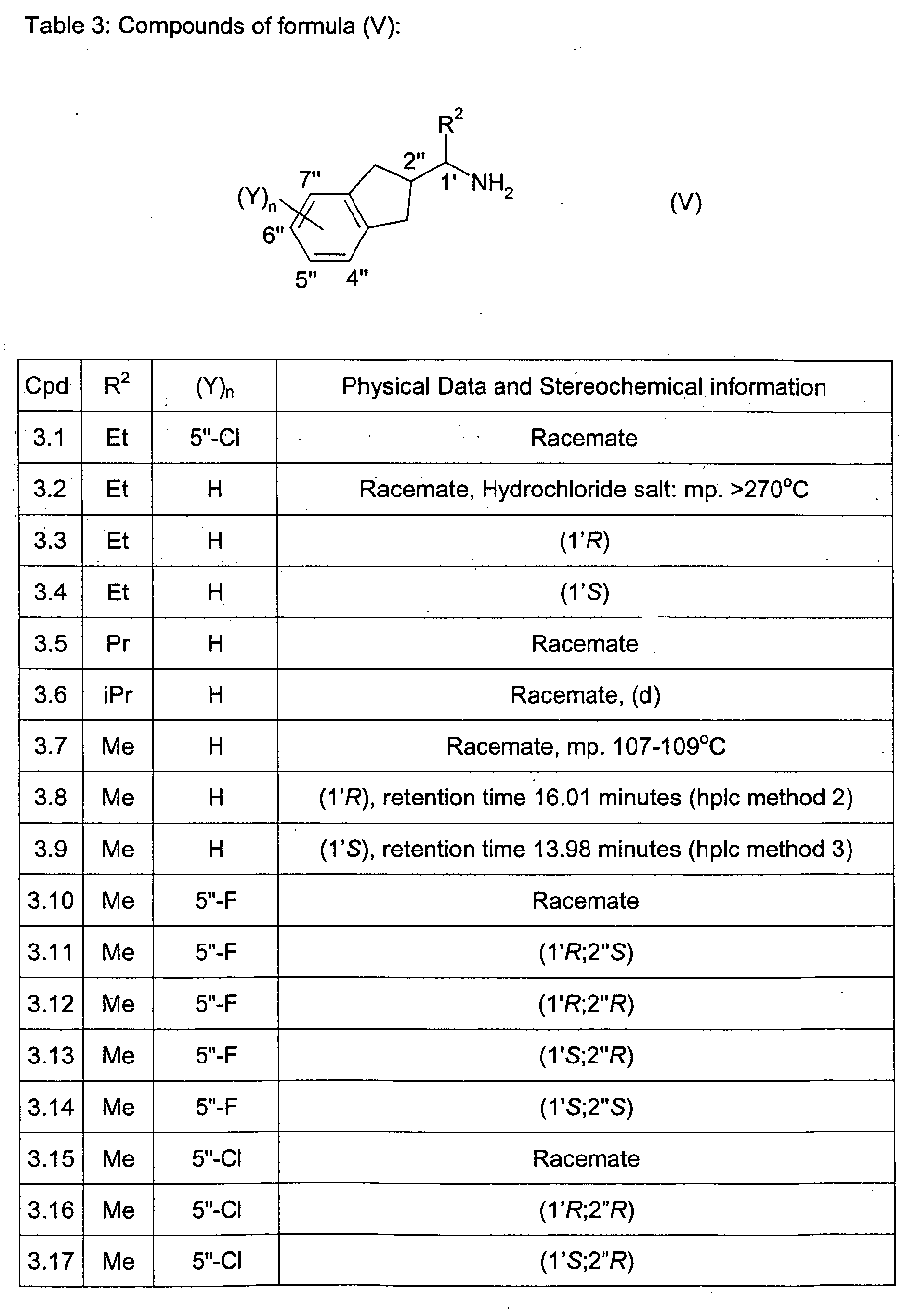

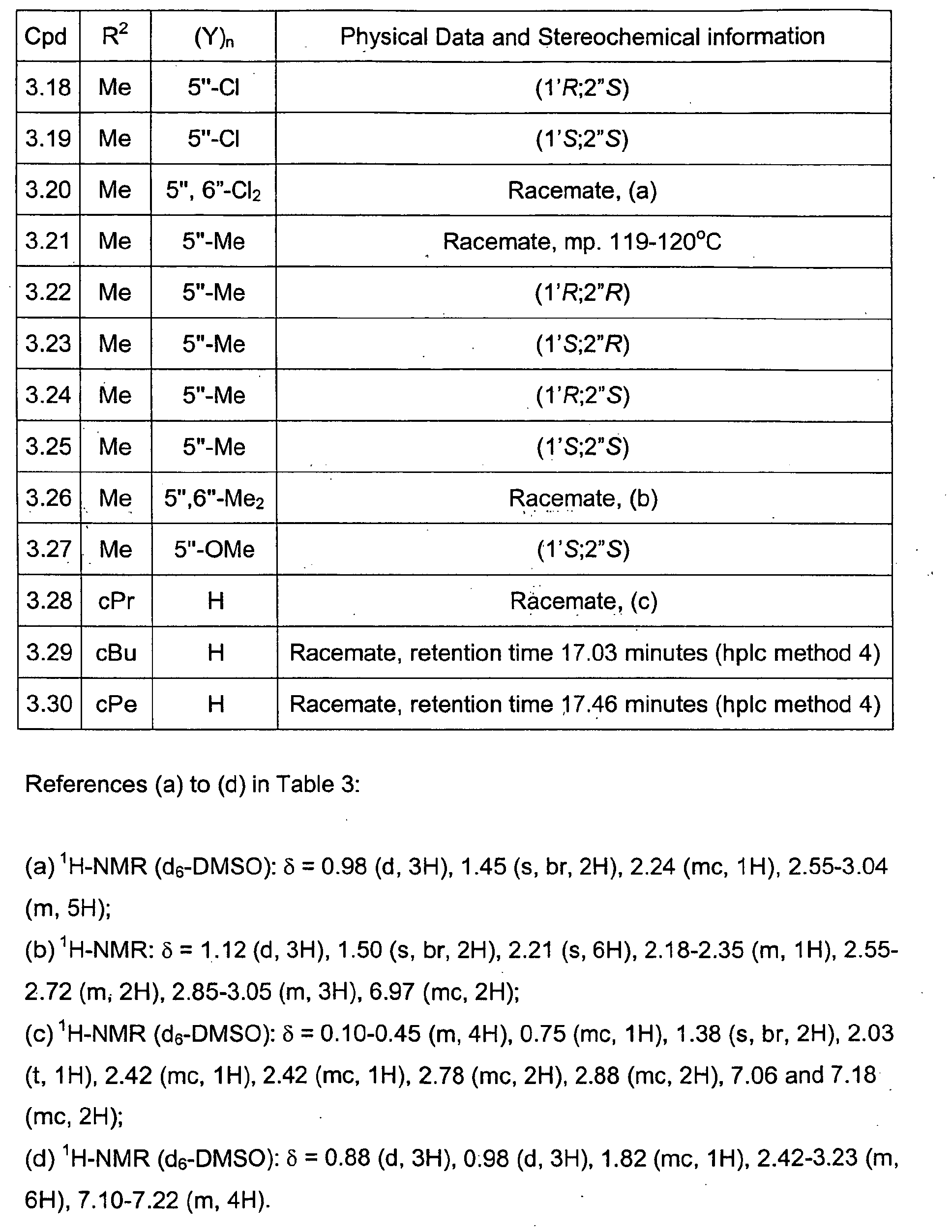

According to a further feature of the present invention, there is provided the use as a herbicide or plant growth regulator characterised in that the compound of formula (I) or a salt thereof is applied in an effective amount for the control of weeds or for regulating the growth of plants at a plant locus. For this purpose, the said compound is normally used in the form of a herbicidal composition (i.e. in association with compatible diluents or carriers and/or surface active agents suitable for use in herbicidal compositions), for example as hereinafter described.
By application to the 'plant locus' is meant application, for example to the plant growing medium, such as soil, as well as to the seeds, emerging seedlings, roots, stems, leaves or other plant parts.

The compounds of the formula (I) and their salts, all termed hereinbelow as compounds of formula (I), have an excellent herbicidal activity against a broad range of economically important monocotyledonous and dicotyledonous harmful plants. The compounds of formula (I) also act efficiently on perennial weeds which produce shoots from rhizomes, root stocks or other perennial organs and which are difficult to control. In this context, it does not matter whether the substances are applied pre-planting, pre-emergence or post-emergence.
Specifically, examples may be mentioned of some representatives of the monocotyledonous and dicotyledonous weed flora which can be controlled by the compounds of formula (I), without the enumeration being a restriction to certain species.

Amongst the monocotyledonous weed species, those on which the active substances act efficiently are, for example, Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea and Cyperus species from the annual group and, amongst the perennial species, Agropyron, Cynodon, Imperata and Sorghum and also perennial Cyperus species.
In the case of dicotyledonous weed species, the spectrum of action extends to species such as, for example, Galium, Viola, Veronica, Lamium, Stellaria, Amaranthus, Sinapis, Ipomoea, Matricaria, Abutilon and Sida amongst the annuals and Convolvulus, Cirsium, Rumex and Artemisia in the case of the perennial weeds. Herbicidal action is also achieved in the case of dicotyledonous harmful plants such as Ambrosia, Anthemis, Carduus, Centaurea, Chenopodium, Cirsium, Convolvulus, Datura, Emex, Galeopsis, Galinsoga, Kochia, Lepidium, Lindernia, Papaver, Portlaca, Polygonum, Ranunculus, Rorippa, Rotala, Seneceio, Sesbania, Solanum, Sonchus, Taraxacum, Trifolium, Urtica and Xanthium.

Harmful plants occurring under the specific cultivation conditions of rice, such as, for example, Sagittaria, Alisma, Eleocharis, Scirpus and Cyperus, are also outstandingly well controlled by the active substances according to the invention.

If the compounds according to the invention are applied to the soil surface before germination, then the weed seedlings are either prevented completely from emerging or the weeds grow until they have reached the cotyledon stage, but then their growth stops and they finally die completely after three to four weeks have elapsed.
When the active substances are applied postemergence to the green parts of the plants, growth stops equally drastically a very short time after treatment and the weed plants remain at the stage of growth at the time of application, or they die completely after a certain time, so that in this manner competition by the weeds, which is harmful to the crop plants, is eliminated at a very early stage and in a sustained manner.

Although the compounds according to the invention have an excellent herbicidal activity against mono- and dicotyledonous weeds, crop plants of economically important crops such as, for example, wheat, barley, rye, triticale, rice, maize, sugar beet, cotton and soybeans (particularly wheat, barley, rice and maize) are damaged only to an insignificant extent or not at all. For these reasons, the present compounds are very highly suitable for the selective control of undesired vegetation in stands of agriculturally useful plants or in stands of ornamental plants.

In addition, the substances according to the invention have outstanding growth-regulatory properties in crop plants. They engage in the plants' metabolism in a regulatory fashion and can thus be employed for influencing plant constituents in a targeted fashion and for facilitating harvesting, such as, for example, by triggering desiccation and stunted growth. Moreover, they are also suitable for generally controlling and inhibiting undesired vegetative growth without simultaneously killing the plants. Inhibiting vegetative growth plays an important role in many monocotyledonous and dicotyledonous crops since lodging can be reduced, or prevented completely, hereby.

Due to their herbicidal and plant-growth regulatory properties, the compounds of formula (I) can also be employed for controlling harmful plants in crops of known genetically modified plants, or genetically modified plants yet to be developed. As a rule, the transgenic plants are distinguished by particular advantageous properties, for example by resistances to certain pesticides, mainly certain herbicides; resistances to plant diseases or pathogens of plant diseases, such as certain insects or microorganisms such as fungi, bacteria or viruses. Other particular properties relate, for example, to the harvested material with regard to quantity, quality, storage properties, composition and specific constituents. Thus, transgenic plants are known where the starch content is increased or the starch quality is altered or those where the harvested material has a different fatty acid spectrum.

The compounds of formula (I) are preferably employed in economically important transgenic crops of useful plants and ornamentals, for example cereals such as wheat, barley, rye, oats, sorghum and millet, rice, cassava and maize, or else crops of sugar beet, cotton, soya, oil seed rape, potatoes, tomatoes, peas and other vegetables.
The compounds of formula (I) can preferably be employed as herbicides in crops of useful plants which are resistant to the phytotoxic effects of the herbicides or have been rendered thus by means of genetic engineering.

Traditional ways of generating novel plants which have modified characteristics in comparison with existing plants consist, for example, in traditional breeding methods and the generation of mutants. However, it is also possible to generate novel plants with altered characteristics with the aid of genetic engineering methods (see, for example, EP-A-0221044, EP-A-0131624). For example, several cases have been described of
- genetic engineering modifications of crop plants with the purpose of modifying the starch synthesized in the plants (for example WO 92/11376, WO 92/14827, WO 91/19806),
- transgenic crop plants which are resistant to certain herbicides of the glufosinate type (cf., for example, EP-A-0242236, EP-A-242246) or the glyphosate type (WO 92/00377) or the sulfonylurea type (EP-A-0257993, US-A-5013659),
- transgenic crop plants, for example cotton, which are capable of producing Bacillus thuringiensis toxins (Bt toxins) which make the plants resistant to specific pests (EP-A-0142924, EP-A-0193259),
- transgenic crop plants whose fatty acid spectrum is modified (WO 91/13972).

A large number of techniques in molecular biology by means of which novel transgenic plants with altered characteristics can be generated are known in principle; see, for example, Sambrook et al., 1989, Molecular Cloning, A Laboratory Manual, 2nd Ed., Cold Spring Harbor Laboratory Press, Cold Spring Harbor, NY; or Winnacker "Gene und Klone" [Genes and Clones], VCH Weinheim 2nd Edition 1996, or Christou, "Trends in Plant Science" 1 (1996) 423-431).

In order to perform such genetic engineering manipulations, nucleic acid molecules may be introduced into plasmids which allow mutagenesis or a sequence change by means of recombination of DNA sequences. It is possible, for example, with the aid of the abovementioned standard methods to perform base exchanges, to remove subsequences or to add natural or synthetic sequences. To connect the DNA fragments to each other, adaptors or linkers may be attached to the fragments.

For example, plant cells with a reduced activity of a gene product can be generated by expressing at least one corresponding antisense RNA, a sense RNA to achieve a cosuppressory effect or by expressing at least one ribozyme of suitable construction which specifically cleaves transcripts of the abovementioned gene product.

To this end it is possible to make use of, on the one hand, DNA molecules which encompass the entire coding sequence of a gene product inclusive of any flanking sequences which may be present, on the other hand DNA molecules which only encompass parts of the coding sequence, but these parts must be long enough in order to effect, in the cells, an antisense effect. Use may also be made of DNA sequences which show a high degree of homology to the coding sequences of a gene product, but which are not completely identical.

When nucleic acid molecules are expressed in plants, the protein which has been synthesized may be located in any desired compartment of the plant cell. However, to achieve localization in a particular compartment, it is possible, for example, to link the coding region with DNA sequences which guarantee localization in a particular compartment. Such sequences are known to the skilled worker (see, for example, Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

The transgenic plant cells may be regenerated by known techniques to give complete plants. In principle, the transgenic plants can be plants of any desired plant species, that is to say monocotyledonous and also dicotyledonous plants.

This allows transgenic plants to be obtained which exhibit altered characteristics by means of overexpression, suppression or inhibition of homologous (= natural) genes or gene sequences or by means of expression of heterologous (= foreign) genes or gene sequences.

The compounds of formula (I) can preferably be employed in transgenic crops which are resistant to herbicides from the group of the sulfonylureas, glufosinate-ammonium or glyphosate-isopropylammonium and analogous active substances.

When the compounds of formula (I) are used in transgenic crops, effects other than the herbicidal effects to be observed in other crops are frequently found which are specific for application in the particular transgenic crop, for example an altered or specifically widened weed spectrum which can be controlled, altered application rates which may be employed for application, preferably good combining ability with the herbicides to which the transgenic crop is resistant, and an effect on growth and yield of the transgenic crop plants.

The invention therefore also relates to the use of the compounds of formula (I) as herbicides for controlling harmful plants in transgenic crop plants.

The use according to the invention for controlling harmful plants or for regulating the growth of plants also includes the case where the compounds of formula (I) are only formed in the plant or the soil from a precursor ("prodrug") after its application to the plant.

The compounds of formula (I) can be employed in the conventional preparations as wettable powders, emulsifiable concentrates, sprayable solutions, dusts or granules. The invention therefore also relates to herbicidal and plant-growth-regulating compositions which comprise compounds of formula (I).

According to a further feature of the present invention, there is provided a herbicidal or plant growth regulating composition comprising an effective amount of a compound of formula (I) as defined above or an agriculturally acceptable salt thereof in association with, and preferably homogeneously dispersed in, one or more compatible agriculturally- acceptable diluents or carriers and/or surface active agents [i.e. diluents or carriers and/or surface active agents of the type generally accepted in the art as being suitable for use in herbicidal compositions and which are compatible with compounds of the invention]. The term "homogeneously dispersed" is used to include compositions in which the compounds of formula (I) are dissolved in other components. The term "herbicidal compositions" is used in a broad sense to include not only compositions which are ready for use as herbicides but also concentrates which must be diluted before use (including tank mixtures).

The compounds of formula (I) can be formulated in various ways, depending on the prevailing biological and/or chemico-physical parameters. Examples of possible formulations which are suitable are: wettable powders (WP), water-soluble powders (SP), water-soluble concentrates, emulsifiable concentrates (EC), emulsions (EW) such as oil-in-water and water-in-oil emulsions, sprayable solutions, suspension concentrates (SC), dispersions on an oil or water basis, solutions which are miscible with oil, capsule suspensions (CS), dusts (DP), seed-dressing products, granules for broadcasting and soil application, granules (GR) in the form of microgranules, spray granules, coated granules and adsorption granules, water-dispersible granules (WG), water-soluble granules (SG), ULV formulations, microcapsules and waxes.

These individual formulation types are known in principle and described, for example, in: Winnacker-Küchler, "Chemische Technologie" [Chemical Technology], Volume 7, C. Hauser Verlag, Munich, 4th Edition 1986; Wade van Valkenburg, "Pesticide Formulations", Marcel Dekker, N.Y., 1973; K. Martens, "Spray Drying Handbook", 3rd Ed. 1979, G. Goodwin Ltd. London.

The necessary formulation auxiliaries such as inert materials, surfactants, solvents and other additives are also known and described, for example, in: Watkins, "Handbook of Insecticide Dust Diluents and Carriers", 2nd Ed., Darland Books, Caldwell N.J.; H.v. Olphen, "Introduction to Clay Colloid Chemistry", 2nd Ed., J. Wiley & Sons, N.Y.; C. Marsden, "Solvents Guide", 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte" [Surface-active ethylene oxide adducts], Wiss. Verlagsgesell., Stuttgart 1976; Winnacker-Küchler, "Chemische Technologie" [Chemical Technology], Volume 7, C. Hauser Verlag, Munich, 4th Ed. 1986.

Based on these formulations, it is also possible to prepare combinations with other pesticidally active substances such as, for example, insecticides, acaricides, herbicides, fungicides, and with safeners, fertilizers and/or growth regulators, for example in the form of a readymix or a tank mix.

Wettable powders are preparations which are uniformly dispersible in water and which, besides the compounds of formula (I), also comprise ionic and/or nonionic surfactants (wetters, dispersants), for example, polyoxyethylated alkylphenols, polyoxyethylated fatty alcohols, polyoxyethylated fatty amines, fatty alcohol polyglycol ether sulfates, alkanesulfonates or alkylbenzenesulfonates, sodium lignosulfonate, sodium 2,2'-dinaphthylmethane-6,6'-disulfonate, sodium dibutylnaphthalenesulfonate or else sodium oleoylmethyltaurinate, in addition to a diluent or inert substance. To prepare the wettable powders, the compounds of formula (I) are, for example, ground finely in conventional apparatuses such as hammer mills, blower mills and air-jet mills and mixed with the formulation auxiliaries either concomitantly or thereafter.

Emulsifiable concentrates are prepared, for example, by dissolving the compounds of formula (I) in an organic solvent, for example butanol, cyclohexanone, dimethylformamide, xylene or else higher-boiling aromatics or hydrocarbons or mixtures of these, with addition of one or more ionic and/or nonionic surfactants (emulsifiers). Emulsifiers which can be used are, for example: calcium salts of alkylarylsulfonic acids, such as calcium dodecylbenzenesulfonate or nonionic emulsifiers, such as fatty acid polyglycol esters, alkylaryl polyglycol ethers, fatty alcohol polyglycol ethers, propylene oxide/ethylene oxide condensates, alkyl polyethers, sorbitan esters such as sorbitan fatty acid esters or polyoxyethylene sorbitan esters such as polyoxyethylene sorbitan fatty acid esters.

Dusts are obtained by grinding the active substance with finely divided solid substances, for example talc or natural clays, such as kaolin, bentonite or pyrophyllite, or diatomaceous earth.

Suspension concentrates may be water- or oil-based. They can be prepared, for example, by wet grinding by means of commercially available bead mills, if appropriate with addition of surfactants, as they have already been mentioned above for example in the case of the other formulation types.

Emulsions, for example oil-in-water emulsions (EW), can be prepared for example by means of stirrers, colloid mills and/or static mixtures using aqueous organic solvents and, if appropriate, surfactants as they have already been mentioned above for example in the case of the other formulation types.

Granules can be prepared either by spraying the compounds of formula (I) onto adsorptive, granulated inert material or by applying active substance concentrates onto the surface of carriers such as sand, kaolinites or of granulated inert material, by means of binders, for example polyvinyl alcohol, sodium polyacrylate or alternatively mineral oils. Suitable active substances can also be granulated in the manner which is conventional for the production of fertilizer granules, if desired in a mixture with fertilizers.

Water-dispersible granules are prepared, as a rule, by the customary processes such as spray-drying, fluidized-bed granulation, disk granulation, mixing in highspeed mixers and extrusion without solid inert material. To prepare disk, fluidized-bed, extruder and spray granules, see, for example, processes in "Spray-Drying Handbook" 3rd ed. 1979, G. Goodwin Ltd., London; J.E. Browning, "Agglomeration", Chemical and Engineering 1967, pages 147 et seq.; "Perry's Chemical Engineer's Handbook", 5th Ed., McGraw-Hill, New York 1973, p. 8-57.

For further details on the formulation of crop protection products, see, for example, G.C. Klingman, "Weed Control as a Science", John Wiley and Sons, Inc., New York, 1961, pages 81-96 and J.D. Freyer, S.A. Evans, "Weed Control Handbook", 5th Ed., Blackwell Scientific Publications, Oxford, 1968, pages 101-103.

As a rule, the agrochemical preparations comprise 0.1 to 99% by weight, in particular 0.1 to 95% by weight, of compounds of formula (I).
The concentration of compounds of formula (I) in wettable powders is, for example, approximately 10 to 90% by weight, the remainder to 100% by weight being composed of customary formulation components. In the case of emulsifiable concentrates, the concentration of compounds of formula (I) can amount to approximately 1 to 90, preferably 5 to 80% by weight. Formulations in the form of dusts usually comprise 1 to 30% by weight of compounds of formula (I), preferably in most cases 5 to 20% by weight of compounds of formula (I), while sprayable solutions comprise approximately 0.05 to 80, preferably 2 to 50% by weight of compounds of formula (I). In the case of water-dispersible granules, the content of compounds of formula (I) depends partly on whether the compounds of formula (I) are in liquid or solid form and on which granulation auxiliaries, fillers and the like are being used. The water-dispersible granules, for example, comprise between 1 and 95% by weight of active substance, preferably between 10 and 80% by weight.

In addition, the formulations of compounds of formula (I) mentioned comprise, if appropriate, the adhesives, wetters, dispersants, emulsifiers, penetrants, preservatives, antifreeze agents, solvents, fillers, carriers, colorants, antifoams, evaporation inhibitors, pH regulators and viscosity regulators which are conventional in each case.

The compounds of the formula (I) or their salts can be employed as such or in the form of their preparations (formulations) as combinations with other pesticidally active substances, such as, for example, insecticides, acaricides, nematicides, herbicides, fungicides, safeners, fertilizers and/or growth regulators, for example as a premix or as tank mixes.
Components which may be employed for the active substances according to the invention in mixed formulations or in tank mix are, for example, known active compounds which are based on an inhibition of, for example, acetolactate synthase, acetyl-coenzyme A carboxylase, PS I, PS II, HPPDO, phytoene desaturase, protoporphyrinogen oxidase, glutamine synthetase, cellulose biosynthesis, 5-enolpyruvylshikimate-3-phosphate synthetase. Such compounds, and also other compounds which can be employed, whose mechanism of action is to a degree unknown or different, are described, for example, in Weed Research 26, 441-445 (1986), or "The Pesticide Manual", 11th Edition 1997 (hereinbelow also abbreviated to "PM") and 12th Edition 2000, The British Crop Protection Council and the Royal Soc. of Chemistry (editors) and literature cited therein. Herbicides which are known from the literature and which can be mentioned, which can be combined with the compounds of the formula (I), are, for example, the following active substances (Note: the compounds are either designated by the common name according to the International Organization for Standardization (ISO) or using the chemical name, if appropriate together with a customary code number):
acetochlor; acifluorfen(-sodium); aclonifen; AKH 7088, i.e. [[[1-[5-[2-chloro-4-(trifluoromethyl)phenoxy]-2-nitrophenyl]-2-methoxyethylidene]amino]oxy]acetic acid and its methyl ester; alachlor; alloxydim(-sodium); ametryn; amicarbazone, amidochlor, amidosulfuron; amitrol; AMS, i.e. ammonium sulfamate; anilofos; asulam; atrazin; azafenidin, azimsulfurone (DPX-A8947); aziprotryn; barban; BAS 516 H, i.e. 5-fluoro-2-phenyl-4H-3,1-benzoxazin-4-one; beflubutamid, benazolin(-ethyl); benfluralin; benfuresate; bensulfuron(-methyl); bensulide; bentazone; benzobicyclon, benzofenap; benzofluor; benzoylprop(-ethyl); benzthiazuron; bialaphos; bifenox; bispyribac(-sodium), bromacil; bromobutide; bromofenoxim; bromoxynil; bromuron; buminafos; busoxinone; butachlor; butafenacil, butamifos; butenachlor; buthidazole; butralin; butroxydim, butylate; cafenstrole (CH-900); carbetamide; carfentrazone(-ethyl) (ICI-A0051); caloxydim, CDAA, i.e. 2-chloro-N,N-di-2-propenylacetamide; CDEC, i.e. 2-chloroallyl diethyldithiocarbamate; chlomethoxyfen; chloramben; chlorazifop-butyl, chlormesulon (ICI-A0051); chlorbromuron; chlorbufam; chlorfenac; chlorflurecolmethyl; chloridazon; chlorimuron(-ethyl); chlornitrofen; chlorotoluron; chloroxuron; chlorpropham; chlorsulfuron; chlorthal-dimethyl; chlorthiamid; chlortoluron, cinidon(-methyl and -ethyl), cinmethylin; cinosulfuron; clefoxydim, clethodim; clodinafop and its ester derivatives (for example clodinafop-propargyl); clomazone; clomeprop; cloproxydim; clopyralid; clopyrasulfuron(-methyl), cloransulam(-methyl), cumyluron (JC 940); cyanazine; cycloate; cyclosulfamuron (AC 104); cycloxydim; cycluron; cyhalofop and its ester derivatives (for example the butyl ester, DEH-112); cyperquat; cyprazine; cyprazole; daimuron; 2,4-D, 2,4-DB; 2,4-DB, dalapon; desmedipham; desmetryn; di-allate; dicamba; dichlobenil; dichlorprop; diclofop and its esters such as diclofop-methyl; diclosulam, diethatyl(-ethyl); difenoxuron; difenzoquat; diflufenican; diflufenzopyr, dimefuron; dimepiperate, dimethachlor; dimethametryn; dimethenamid (SAN-582H); dimethazone, dimexyflam, dimethipin; dimetrasulfuron, dinitramine; dinoseb; dinoterb; diphenamid; dipropetryn; diquat; dithiopyr; diuron; DNOC; eglinazine-ethyl; EL 77, i.e.
5-cyano-1-(1,1-dimethylethyl)-N-methyl-1H-pyrazole-4-carboxamide; endothal; epoprodan, EPTC; esprocarb; ethalfluralin; ethametsulfuron-methyl; ethidimuron; ethiozin; ethofumesate; ethoxyfen and its esters (for example the ethyl ester, HN-252); ethoxysulfuron, etobenzanid (HW 52); F5231, i.e. N-[2-chloro-4-fluoro-5-[4-(3-fluoropropyl)-4,5-dihydro-5-oxo-1H-tetrazol-1-yl]phenyl]ethanesulfon amide; fenoprop; fenoxan, fenoxaprop and fenoxaprop-P and their esters, for example fenoxaprop-P-ethyl and fenoxaprop-ethyl; fenoxydim; fentrazamide, fenuron; flamprop(-methyl or -isopropyl or -isopropyl-L); flazasulfuron; floazulate, florasulam, fluazifop and fluazifop-P and their esters, for example fluazifop-butyl and fluazifop-P-butyl; flucarbazone(-sodium), fluchloralin; flumetsulam; flumeturon; flumiclorac(-pentyl), flumioxazin (S-482); flumipropyn; fluometuron, fluorochloridone, fluorodifen; fluoroglycofen(-ethyl); flupoxam (KNW-739); flupropacil (UBIC-4243); flupyrsulfuron(-methyl or -sodium), flurenol(-butyl), fluridone; flurochloridone; fluroxypyr(-meptyl); flurprimidol, flurtamone; fluthiacet(-methyl), fluthiamide, fomesafen; foramsulfuron, fosamine; furyloxyfen; glufosinate(-ammonium); glyphosate(-isopropylammonium); halosafen; halosulfuron(-methyl) and its esters (for example the methyl ester, NC-319); haloxyfop and its esters; haloxyfop-P (= R-haloxyfop) and its esters; hexazinone; imazamethabenz(-methyl); imazapyr; imazaquin and salts such as the ammonium salt; imazamethapyr, imazamox, imazapic, imazethamethapyr; imazethapyr; imazosulfuron; indanofan, iodosulfuron(-methyl-sodium), ioxynil; isocarbamid; isopropalin; isoproturon; isouron; isoxaben; isoxachlortole, isoxaflutole, isoxapyrifop; karbutilate; lactofen; lenacil; linuron; MCPA; MCPB; mecoprop; mefenacet; mefluidid; mesosulfuron, mesotrione, metamitron; metazachlor; methabenzthiazuron; metham; methazole; methoxyphenone; methyldymron; metabenzuron, methobenzuron; metobromuron; (alpha-)metolachlor; metosulam (XRD 511); metoxuron; metribuzin; metsulfuron-methyl; MH; molinate; monalide; monocarbamide dihydrogensulfate; monolinuron; monuron; MT 128, i.e.
6-chloro-N-(3-chloro-2-propenyl)-5-methyl-N-phenyl-3-pyridazinamine; MT 5950, i.e. N-[3-chloro-4-(1-methylethyl)-phenyl]-2-methylpentanamide; naproanilide; napropamide; naptalam; NC 310, i.e. 4-(2,4-dichlorobenzoyl)-1-methyl-5-benzyloxypyrazole; neburon; nicosulfuron; nipyraclophen; nitralin; nitrofen; nitrofluorfen; norflurazon; orbencarb; oryzalin; oxadiargyl (RP-020630); oxadiazon; oxasulfuron, oxaziclomefone, oxyfluorfen; paraquat; pebulate; pelargonic acid, pendimethalin; pentoxazone, perfluidone; phenisopham; phenmedipham; picloram; picolinafen, piperophos; piributicarb; pirifenop-butyl; pretilachlor; primisulfuron(-methyl); procarbazone-(sodium), procyazine; prodiamine; profluralin; proglinazine(-ethyl); prometon; prometryn; propachlor; propanil; propaquizafop and its esters; propazine; propham; propisochlor; propyzamide; prosulfalin; prosulfocarb; prosulfuron (CGA-152005); prynachlor; pyraflufen(-ethyl), pyrazolinate; pyrazon; pyrazosulfuron(-ethyl); pyrazoxyfen; pyribenzoxim, pyributicarb, pyridafol, pyridate; pyrimidobac(-methyl), pyrithiobac(-sodium) (KIH-2031); pyroxofop and its esters (for example the propargyl ester); quinclorac; quinmerac; quinoclamine, quinofop and its ester derivatives, quizalofop and quizalofop-P and their ester derivatives, for example quizalofop-ethyl; quizalofop-P-tefuryl and -ethyl; renriduron; rimsulfuron (DPX-E 9636); S 275, i.e. 2-[4-chloro-2-fluoro-5-(2-propynyloxy)phenyl]-4,5,6,7-tetrahydro-2H-indazole; secbumeton; sethoxydim; siduron; simazine; simetryn; SN 106279, i.e. 2-[[7-[2-chloro-4-(trifluoromethyl)phenoxy]-2-naphthalenyl]oxy]propanoic acid and its methyl ester; sulcotrione, sulfentrazon (FMC-97285, F-6285); sulfazuron; sulfometuron(-methyl); sulfosate (ICI-A0224); sulfosulfuron, TCA; tebutam (GCP-5544); tebuthiuron; tepraloxydim, terbacil; terbucarb; terbuchlor; terbumeton; terbuthylazine; terbutryn; TFH 450, i.e.
N,N-diethyl-3-[(2-ethyl-6-methylphenyl)-sulfonyl]-1H-1,2,4-triazole-1-carboxamide; thenylchlor (NSK-850); thiafluamide, thiazafluron; thiazopyr (Mon-13200); thidiazimin (SN-24085); thifensulfuron(-methyl); thiobencarb; tiocarbazil; tralkoxydim; tri-allate; triasulfuron; triaziflam, triazofenamide; tribenuron(-methyl); triclopyr; tridiphane; trietazine; trifluralin; triflusulfuron and its esters (for example the methyl ester, DPX-66037); trimeturon; tritosulfuron, tsitodef; vernolate; WL 110547, i.e. 5-phenoxy-1-[3-(trifluoromethyl)phenyl]-1H-tetrazole; BAY MKH 6561, UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 and KIH-2023.

Controlling harmful plants selectively is of particular interest in crops of useful plants and ornamentals. Even though the compounds (I) already exhibit very good to sufficient selectivity in many crops, it is possible, in principle, that symptoms of phytotoxicity occur on the cultivated plants in some crops and especially also in the case of mixtures with other herbicides which are less selective. In this respect, combinations of compounds (I) according to the invention which are of particular interest are those which contain the compounds (I) or their combinations with other herbicides or pesticides and safeners. The safeners, which are employed in such an amount that they act as antidote, reduce the phytotoxic side effects of the herbicides/pesticides employed, for example in economically important crops such as cereals (wheat, barley, rye, maize, rice, sorghum and millet), sugar beet, sugar cane, oilseed rape, cotton and soybeans, preferably cereals. The following groups of compounds are examples of suitable safeners for the compounds (I) and their combinations with further pesticides:
a) compounds of the dichlorophenylpyrazoline-3-carboxylic acid type, preferably compounds such as ethyl 1-(2,4-dichlorophenyl)-5- (ethoxycarbonyl)-5-methyl-2-pyrazoline-3-carboxylate (S1-1) ("Mefenpyr-diethyl", PM, pp. 781-782) and related compounds as they are described in WO 91/07874;
b) dichlorophenylpyrazolecarboxylic acid derivatives, preferably compounds such as ethyl 1-(2,4-dichlorophenyl)-5-methylpyrazole-3-carboxylate (S1-2), ethyl 1-(2,4-dichlorophenyl)-5-isopropylpyrazole-3-carboxylate (S1-3), ethyl 1-(2,4-dichlorophenyl)-5-(1,1-dimethylethyl)pyrazole-3-carboxylate (S1-4), ethyl 1-(2,4-dichlorophenyl)-5-phenylpyrazole-3-carboxylate (S1-5) and related compounds as they are described in EP-A-333 131 and EP-A-269 806;
c) compounds of the triazolecarboxylic acids type, preferably compounds such as fenchlorazol (and its ethyl ester), i.e. ethyl 1-(2,4-dichlorophenyl)-5-trichloromethyl-(1H)-1,2,4-triazole-3-carboxylate (S1-6), and related compounds (see EP-A-174 562 and EP-A-346 620);
d) compounds of the 5-benzyl- or 5-phenyl-2-isoxazoline-3-carboxylic acid type or the 5,5-diphenyl-2-isoxazoline-3-carboxylic acid, preferably compounds such as ethyl 5-(2,4-dichlorobenzyl)-2-isoxazoline-3-carboxylate (S1-7) or ethyl 5-phenyl-2-isoxazoline-3-carboxylate (S1-8) and related compounds as they are described in WO 91/08202, or ethyl 5,5-diphenyl-2-isoxazolinecarboxylate (S1-9) ("isoxadifen-ethyl") or n-propyl 5,5-diphenyl-2-isoxazolinecarboxylate (S1-10) or ethyl 5-(4-fluorophenyl)-5-phenyl-2-isoxazoline-3-carboxylate (S1-11), as they are described in German Patent Application (WO-A-95/07897);
e) compounds of the 8-quinolinoxyacetic acid type (S2), preferably 1-methylhex-1-yl (5-chloro-8-quinolinoxy)acetate (common name "cloquintocet-mexyl") (S2-1) (see PM, pp. 263-264) 1,3-dimethylbut-1-yl (5-chloro-8-quinolinoxy)acetate (S2-2), 4-allyloxybutyl (5-chloro-8-quinolinoxy)acetate (S2-3), 1-allyloxyprop-2-yl (5-chloro-8-quinolinoxy)acetate (S2-4), ethyl (5-chloro-8-quinolinoxy)acetate (S2-5), methyl (5-chloro-8-quinolinoxy)acetate (S2-6), allyl (5-chloro-8-quinolinoxy)acetate (S2-7), 2-(2-propylideneiminooxy)-1-ethyl (5-chloro-8-quinolinoxy)acetate (S2-8), 2-oxoprop-1-yl (5-chloro-8-quinolinoxy)acetate (S2-9), and related compounds as are described in EP-A-86 750, EP-A-94 349 and EP-A-191 736 or EP-A-0 492 366;
f) compounds of the (5-chloro-8-quinolinoxy)malonic acid type, preferably compounds such as diethyl (5-chloro-8-quinolinoxy)malonate, diallyl (5-chloro-8-quinolinoxy)malonate, methylethyl (5-chloro-8-quinolinoxy)malonate and related compounds as are described in EP-A-0 582 198;
g) active substances of the phenoxyacetic or phenoxypropionic acid derivatives type or of the aromatic carboxylic acids type, such as, for example, 2,4-dichlorophenoxyacetic acids (and its esters) (2,4-D), 4-chloro-2-methylphenoxypropionic esters (mecoprop), MCPA or 3,6-dichloro-2-methoxybenzoic acid (and its esters) (dicamba);
h) active substances of the pyrimidines type which are employed in rice as soil-acting safeners, such as, for example, "fenclorim" (PM, pp. 511-512) (= 4,6-dichloro-2-phenylpyrimidine), which is also known as safener for pretilachlor in seeded rice;
i) active substances of the dichloroacetamides type, which are frequently employed as pre-emergence safeners (soil-acting safeners), such as, for example,
   "dichlormid" (PM, pp. 363-364) (= N,N-diallyl-2,2-dichloroacetamide),
   "R-29148" (= 3-dichloroacetyl-2,2,5-trimethyl-1,3-oxazolidine, by Stauffer), "benoxacor" (PM, pp. 102-103) (= 4-dichloroacetyl-3,4-dihydro-3-methyl-2H-1,4-benzoxazine),
   "PPG-1292" (= N-allyl-N-[(1,3-dioxolan-2-yl)methyl]dichloroacetamide by PPG Industries),
   "DK-24" (= N-allyl-N-[(allylaminocarbonyl)methyl]dichloroacetamide by Sagro-Chem),
   "AD-67" or "MON 4660" (= 3-dichloroacetyl-1-oxa-3-azaspiro[4,5]decane by Nitrokemia and Monsanto, respectively),
   "diclonon" or "BAS145138" or "LAB145138" (= 3-dichloroacetyl-2,5,5-trimethyl-1,3-diazabicyclo[4.3.0]nonane by BASF) and
   "furilazol" or "MON 13900" (see PM, 637-638) (= (RS)-3-dichloroacetyl-5-(2-furyl)-2,2-dimethyloxazolidine);
j) active substances of the dichloroacetone derivatives type, such as, for example,
   "MG 191" (CAS Reg. No. 96420-72-3) (= 2-dichloromethyl-2-methyl-1,3-dioxolane by Nitrokemia), which is known as safener for maize;
k) active substances of the oxyimino compounds type, which are known as seed treatment products, such as, for example,
   "oxabetrinil" (PM, pp. 902-903) (= (Z)-1,3-dioxolan-2-ylmethoxyimino(phenyl)-acetonitrile), which is known as seed-treatment safener for sorghum and millet against metolachlor damage,
   "fluxofenim" (PM, pp. 613-614) (= 1-(4-chlorophenyl)-2,2,2-trifluoro-1-ethanone O-(1,3-dioxolan-2-ylmethyl)oxime, which is known as seed-dressing safener for sorghum and millet against metolachlor damage, and
   "cyometrinil" or "-CGA-43089" (PM, p. 1304) (= (Z)-cyanomethoxyimino(phenyl)acetonitrile), which is known as seed-treatment safener for sorghum and millet against metolachlor damage;
l) active substances of the thiazolecarboxylic ester type, which are known as seed treatment products, such as, for example,
   "flurazol" (PM, pp. 590-591) (= benzyl 2-chloro-4-trifluoromethyl-1,3-thiazole-5-carboxylate), which is known as seed-treatment safener for sorghum and millet against alachlor and metolachlor damage;
m) active substances of the naphthalenedicarboxylic acid derivatives type, which are known as seed treatment products, such as, for example,
   "naphthalic anhydride" (PM, p. 1342) (= 1,8-naphthalenedicarboxylic anhydride), which is known as seed-treatment safener for maize against thiocarbamate herbicide damage;
n) active substances of the chromanacetic acid derivatives type, such as, for example,
   "CL 304415" (CAS Reg. No. 31541-57-8) (= 2-(4-carboxychroman-4-yl)acetic acid by American Cyanamid), which is known as safener for maize against damage by imidazolinones;
o) active substances which, in addition to a herbicidal action against harmful plants, also exhibit a safener action in connection with crop plants such as rice, such as, for example,
   "dimepiperate" or "MY-93" (PM, pp. 404-405) (= S-1-methyl-1-phenylethyl piperidine-1-carbothioate), which is known as safener for rice against damage by the herbicide molinate,
   "daimuron" or "SK 23" (PM, p. 330) (= 1-(1-methyl-1-phenylethyl)-3-p-tolylurea), which is known as safener for rice against damage by the herbicide imazosulfuron,
   "cumyluron" = "JC-940" (= 3-(2-chlorophenylmethyl)-1-(1-methyl-1-phenylethyl)urea, see JP-A-60087254), which is known as safener for rice against damage by several herbicides,
   "methoxyphenone" or "NK 049" (= 3,3'-dimethyl-4-methoxybenzophenone), which is known as safener for rice against damage by several herbicides,
   "CSB" (= 1-bromo-4-(chloromethylsulfonyl)benzene) (CAS Reg. No. 54091-06-4, by Kumiai), which is known as safener in rice against damage by several herbicides;
p) N-acylsulfonamides of the formula (S3) and their salts 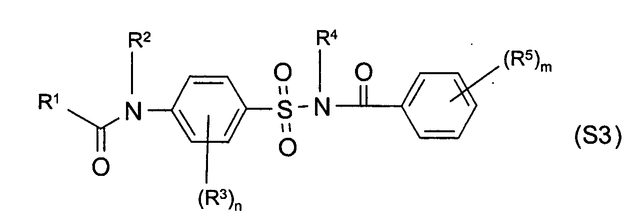 as are described in WO-A-97/45016;
q) acylsulfamoylbenzamides of the formula (S4), if appropriate also in salt form, 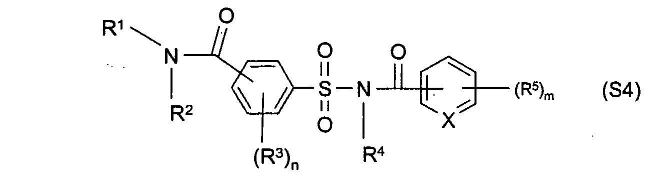 as are described in International Application No. PCT/EP98/06097; and
r) compounds of the formula (S5), 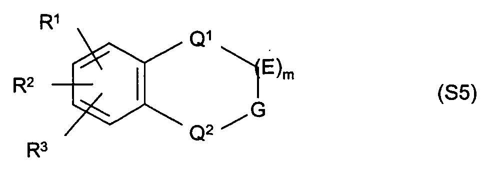
as are described in WO-A 98/13 361,
including the stereoisomers and the salts conventionally used in agriculture.

Amongst the safeners mentioned, those which are of particular interest are (S1-1) and (S1-9) and (S2-1), in particular (S1-1) and (S1-9).
Some of the safeners are already known as herbicides and therefore simultaneously also display a protective action in connection with the crop plants in addition to the herbicidal action in connection with harmful plants.

The weight ratio of herbicide (mixture) to safener generally depends on the application rate of herbicide and the efficacy of the safener in question; it can vary within wide limits, for example in the range of from 200:1 to 1:200, preferably from 100:1 to 1:100, in particular 20:1 to 1:20. The safeners can be formulated with further herbicides/pesticides, analogously to the compounds (I) or their mixtures, and provided and used as readymix or tank mix together with the herbicides.

For use, the herbicide or herbicide safener formulations, which are present in a customary commercial form, are, if appropriate, diluted in the customary fashion, for example using water in the case of wettable powders, emulsifiable concentrates, dispersions and water-dispersible granules. Preparations in the form of dusts, soil granules, granules for spreading, and sprayable solutions, are usually not diluted further with other inert materials prior to use.

The application rate required of the compounds of the formula (I) varies with, inter alia, the external conditions such as temperature, humidity and the type of the herbicide used. It can vary within wide limits, for example between 0.001 and 10.0 kg/ha or more of active substance, but it is preferably between 0.002 and 3 kg/ha, in particular 0.005 and 1 kg/ha.

### B. Formulation examples

a) A dust is obtained by mixing 10 parts by weight of a compound of formula (I) and 90 parts by weight of talc as inert material and grinding the mixture in a hammer mill.
b) A wettable powder which is readily dispersible in water is obtained by mixing 25 parts by weight of a compound of formula (I), 64 parts by weight of kaolin-containing quartz as inert material, 10 parts by weight of potassium lignosulfonate and 1 part by weight of sodium oleoylmethyltaurinate as wetter and dispersant and grinding the mixture in a pinned-disk mill.
c) A dispersion concentrate which is readily dispersible in water is obtained by mixing 20 parts by weight of a compound of formula (I) with 6 parts by weight of alkylphenol polyglycol ether (®Triton X 207), 3 parts by weight of isotridecanol polyglycol ether (8 EO) and 71 parts by weight of paraffinic mineral oil (boiling range for example approx. 255 to above 277°C) and grinding the mixture in a ball mill to a fineness of below 5 microns.
d) An emulsifiable concentrate is obtained from 15 parts by weight of a compound of formula (I), 75 parts by weight of cyclohexanone as solvent and 10 parts by weight of oxethylated nonylphenol as emulsifier.
e) Water-dispersible granules are obtained by mixing
   75 parts by weight of a compound of formula (I),
   10 parts by weight of calcium ligno-sulfonate,
   5 parts by weight of sodium laurylsulfate,
   3 parts by weight of polyvinyl alcohol and
   7 parts by weight of kaolin,
   grinding the mixture in a pinned disk mill and granulating the powder in a fluidized bed by spraying on water as granulation liquid.
f) Alternatively, water-dispersible granules are obtained by homogenizing and precomminuting, on a colloid mill,
   25 parts by weight of a compound of formula (I),
   5 parts by weight of sodium 2,2'-dinaphthylmethane-6,6'-disulfonate,
   2 parts by weight of sodium oleoylmethyltaurinate,
   1 part by weight of polyvinyl alcohol,
   17 parts by weight of calcium carbonate and
   50 parts by weight of water,
   subsequently grinding the mixture on a bead mill and atomizing and drying the resulting suspension in a spray tower by means of a single-substance nozzle.

### C. Biological examples

### Biological Example 1: Pre-emergence effect on weeds

Seeds or rhizome pieces of monocotyledonous and dicotyledonous weed plants were placed in sandy loam in plastic pots and covered with soil. The compounds according to the invention, which were formulated in the form of wettable powders or emulsion concentrates, were then applied to the surface of the soil cover as aqueous suspension or emulsion at various dosages with an application rate of 600 to 800 I of water per ha (converted).
After the treatment, the pots were placed into a greenhouse and kept under good growth conditions for the weeds. The plant or emergence damage was scored visually after the test plants have emerged after an experimental time of 3 to 4 weeks by comparison with untreated controls.
Compound numbers 1.16, 1.25, 1.36, 1.38, 1.52, 1.53, 1.58, 1.59, 1.60, 1.61, 1.62, 1.63, 1.64, 1.65, 1.66, 1.88, 1.89, 1.90, 1.105, 1.117, 1.118, 1.119, 1.120, 1.121, 1.122, 1.123, 1.152, 1.153, 2.1, 2.3, 2.4 and 2.5 according to the invention show a very good pre-emergence control of harmful plants such as Stellaria media, Lolium multiforum, Amaranthus retroflexus, Sinapis alba, Avena sativa and Setaria viridis when applied at an application rate of 1 kg or less of active ingredient per hectare.

### Biological Example 2: Post-emergence effect on weeds

Seeds or rhizome pieces of monocotyledonous and dicotyledonous weeds were placed in sandy loam in plastic pots, covered with soil and grown in the greenhouse under good growth conditions. Three weeks after sowing, the test plants were treated in the three-leaf stage. Various dosages of the compounds according to the invention, which were formulated as wettable powders or emulsion concentrates, were sprayed to the green plant parts at an application rate of 600 to 800 I of water per ha (converted). After the test plants have been left to stand in the greenhouse for approx. 3 to 4 weeks under optimal growth conditions, the effect of the preparations was scored visually by comparison with untreated controls. Compound numbers 1.16, 1.25, 1.36, 1.38, 1.52, 1.53, 1.58, 1.59, 1.60, 1.61, 1.62, 1.63, 1.64, 1.65, 1.66, 1.88, 1.89, 1.90, 1.105, 1.117, 1.118, 1.119, 1.120, 1.121, 1.122, 1.123, 1.152, 1.153, 2.1, 2.3, 2.4 and 2.5 according to the invention show a very good herbicidal post-emergence activity against against harmful plants such as Sinapis alba, Echinochloa crus-galli, Lolium multiflorum, Stellaria media, Cyperus iria, Amaranthus retroflexus, Setaria viridis, Avena sativa, Lamium purpureum, Matricaria inodora, Papaver rhoeas, Veronica persica, Viola trocolor, Kochia spp and Chenopodium album when applied at an application rate of 1 kg or less of active ingredient per hectare.

### Biological Example 3: Action against harmful plants in rice

Transplanted and seeded rice and typical rice weeds (broad-leaved and grass weeds) were grown in the greenhouse until they had reached the three-leaf stage (Echinochloa crus-galli 1.5-leaves) under paddy rice conditions (flooding level of the water: 2 - 3 cm) in closed plastic pots. They were then treated with the compounds according to the invention. To this end, the formulated active substances were suspended, dissolved or emulsified in water and applied to the paddy water of the test plants at various dosages by application by pouring. After the treatment had been carried out in this way, the test plants were placed into the greenhouse under optimal growth conditions and kept in this way over the entire experimental period. Approximately three weeks after application, the experiments were evaluated by visually scoring the plant damage in comparison with untreated controls. Compound numbers 1.16, 1.25, 1.36, 1.38, 1.52, 1.53, 1.58, 1.59, 1.60, 1.61, 1.62, 1.63, 1.64, 1.65, 1.66, 1.88, 1.89, 1.90, 1.105, 1.117, 1.118, 1.119, 1.120, 1.121, 1.122, 1.123, 1.152, 1.153, 2.1, 2.3, 2.4 and 2.5 show a very good herbicidal action against harmful plants such as, for example, Cyperus monti, Echinochloa crus-galli and Sagittaria pygmaea when applied at an application rate of 1 kg or less of active ingredient per hectare.

### Biological Example 4: Tolerance by crop plants

In further greenhouse experiments seeds of a larger number of crop plants and weeds are placed in sandy loam covered with soil. Some of the pots are treated immediately as described in Biological Example 1, while the remaining pots are placed in the greenhouse until the plants have developed two to three leaves and are then sprayed with various dosages of the substances of formula (I) according to the invention as described in Biological Example 2. Four to five weeks after the application and standing time in the greenhouse, it is found by means of visual scoring that the compounds according to the invention leave dicotyledonous crops such as, for example, soybeans, cotton, oilseed rape, sugar beet and potatoes unharmed when applied pre- and post-emergence, even at high rates of active substance. In addition, some substances also leave graminaceous crops such as, for example, barley, wheat, rye, sorghum, maize or rice, unharmed. The compounds of formula. (I) show an improved degree of selectivity compared to the prior art, and are therefore suitable for controlling undesired vegetation in agricultural crops.

## Claims

1. A compound of the formula (I): 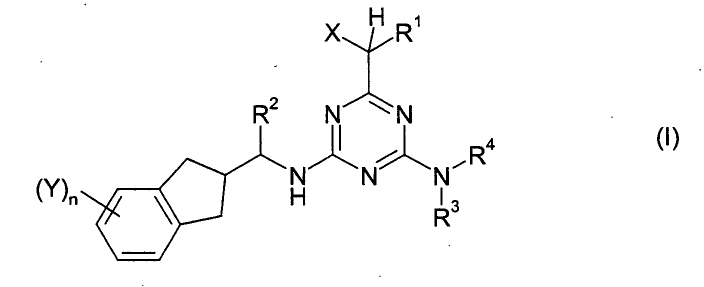 in which:
R¹ is (C₁-C₆)alkyl, (C₁-C₆)haloalkyl, [(C₁-C₄)alkoxy](C₁-C₆)alkyl, (C₂-C₆)alkenyl, (C₂-C₆)haloalkenyl, (C₄-C₆)cycloalkenyl, (C₄-C₆)halocycloalkenyl, (C₁-C₆)alkoxy or (C₁-C₆)haloalkoxy; or is (C₃-C₆)cycloalkyl or (C₃-C₆)halocycloalkyl which cycloalkyl groups are unsubstituted or substituted by one or two (C₁-C₄)alkyl groups;
R² is (C₁-C₄)alkyl, (C₁-C₄)haloalkyl, (C₃-C₆)cycloalkyl or (C₃-C₆)halocycloalkyl;
X is halogen or OH;
R³ and R⁴ are each independently H, (C₁-C₄)alkyl, (C₁-C₄)haloalkyl, (C₃-C₄)alkenyl, (C₃-C₄)haloalkenyl, (C₃-C₄)alkynyl, (C₃-C₄)haloalkynyl or an acyl radical; or
R³ and R⁴ together with the attached nitrogen atom form a five, six or seven membered saturated heterocyclic ring in which one of the ring carbon atoms may be replaced by a hetero atom selected from N, O and S, and which ring is unsubstituted or substituted by (C₁-C₄)alkyl; and
(Y)ₙ is n radicals Y where each Y is independently (C₁-C₄)alkyl, (C₁-C₃)haloalkyl, halogen, (C₁-C₃)alkoxy, (C₁-C₃)haloalkoxy or CN; and
n is 0, 1, 2, 3 or 4; or an agriculturally acceptable salt thereof.

2. A compound or a salt thereof as claimed in claim 1, wherein R¹ is (C₁-C₄)alkyl, (C₁-C₃)haloalkyl, [(C₁-C₂)alkoxy](C₁-C₃)alkyl, (C₂-C₃)alkenyl, (C₂-C₃)haloalkenyl, (C₅-C₆)cycloalkenyl, (C₅-C₆)halocycloalkenyl, (C₁-C₃)alkoxy or (C₁-C₃)haloalkoxy; or is (C₃-C₅)cycloalkyl or (C₃-C₅)halocycloalkyl which cycloalkyl groups are unsubstituted or substituted by one or two methyl or ethyl groups.

3. A compound or a salt thereof as claimed in claim 1 or 2, wherein R² is (C₁-C₄)alkyl, (C₁-C₃)haloalkyl, (C₃-C₅)cycloalkyl or (C₃-C₅)halocycloalkyl.

4. A compound or a salt thereof as claimed in any of claims 1 to 3, wherein X is Cl, F or OH.

5. A compound or a salt thereof as claimed in one of claims 1 to 4, wherein R³ is H, (C₁-C₄)alkyl, (C₁-C₄)haloalkyl, (C₃-C₄)alkenyl, (C₃-C₄)alkynyl or an acyl radical having 1 to 12 carbon atoms.

6. A compound or a salt thereof as claimed in one of claims 1 to 5, wherein R⁴ is H, (C₁-C₄)alkyl or (C₁-C₄)haloalkyl.

7. A compound or a salt thereof as claimed in one of claims 1 to 6, wherein (Y)ₙ is 0, 1 or 2 radicals Y where each Y is independently (C₁-C₃)alkyl, halogen or (C₁-C₃)alkoxy.

8. A compound or a salt thereof as claimed in claim 1, wherein:
R¹ is (C₁-C₄)alkyl, (C₁-C₃)haloalkyl, [(C₁-C₂)alkoxy](C₁-C₃)alkyl, (C₂-C₃)alkenyl, (C₂-C₃)haloalkenyl, (C₅-C₆)cycloalkenyl, (C₅-C₆)halocycloalkenyl, (C₁-C₃)alkoxy or (C₁-C₃)haloalkoxy; or is (C₃-C₅)cycloalkyl or (C₃-C₅)halocycloalkyl which cycloalkyl groups are unsubstituted or substituted by one or two methyl or ethyl groups;
R² is (C₁-C₄)alkyl, (C₁-C₃)haloalkyl, (C₃-C₅)cycloalkyl or (C₃-C₅)halocycloalkyl;
X is halogen or OH;
R³ is H, (C₁-C₄)alkyl, (C₁-C₄)haloalkyl, (C₃-C₄)alkenyl, (C₃-C₄)alkynyl or an acyl radical having 1 to 12 carbon atoms;
R⁴ is H, (C₁-C₄)alkyl or (C₁-C₄)haloalkyl; and
(Y)ₙ is 0, 1 or 2 radicals Y where each Y is independently (C₁-C₃)alkyl, halogen or (C₁-C₃)alkoxy.

9. A process for the preparation of a compound of the formula (I) or a salt thereof as defined in any of claims 1 to 8, which process comprises:
a) where R¹, R², R³, R⁴, X, Y and n are as defined in formula (I), reacting a compound of formula (II): 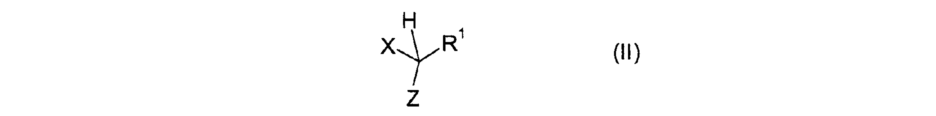 wherein R¹ and X are as defined in formula (I), and Z is a functional group selected from the group consisting of carboxylic ester, carboxylic orthoester, carboxylic acid chloride, carboxamide, cyano, carboxylic anhydride or trichloromethyl, with a biguanidine compound of formula (III) or an acid addition salt thereof: 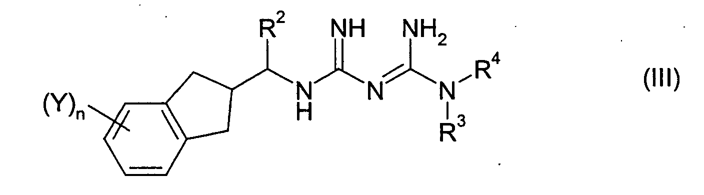 wherein R², R³, R⁴, Y and n are as defined in formula (I);
b) where R¹, R², R³, R⁴, X, Y and n are as defined in formula (I), reacting a compound of formula (IV): 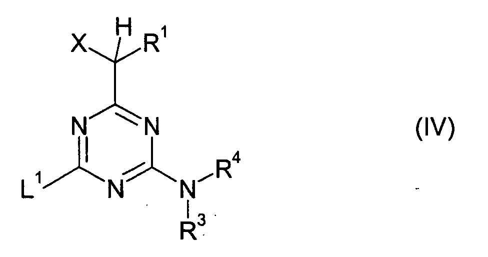 wherein R¹, R³, R⁴ and X are as defined in formula (I), and L¹ is a leaving group, with an amine of formula (V) or an acid addition salt thereof: 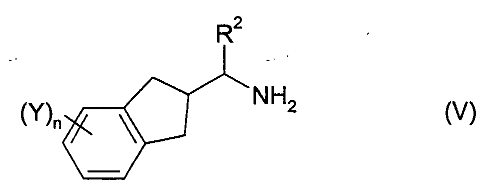 wherein R², Y and n are as defined in formula (I);
c) where R¹, R², X, Y and n are as defined in formula (I), and one of R³ or R⁴ is (C₁-C₄)alkyl or (C₁-C₄)haloalkyl, and the other of R⁴ or R³ respectively is as defined in formula (I), the alkylation of the corresponding compound of formula (I) wherein R³ or R⁴ respectively is H, with an alkylating agent of formula (VI) or (VII) respectively:
R³-L² (VI)
R⁴-L² (VII)
wherein R³ and R⁴ are each (C₁-C₄)alkyl, (C₁-C₄)haloalkyl, and L² is a leaving group; or
d) where R¹, R², X, Y and n are as defined in formula (I), one of R³ or R⁴ is an acyl radical, and the other of R⁴ or R³ respectively is as defined in formula (I), the reaction of the corresponding compound of formula (I) wherein R³ or R⁴ respectively is H, with an acylating agent of formula (VIII) or (IX) respectively:
R³-L³ (VII)
R⁴-L³ (IX)
wherein R³ and R⁴ are each an acyl radical as defined in formula (I) and L³ is a leaving group, or with a formylating agent.

10. A herbicidal or plant growth regulating composition, which comprises one or more compounds of the formula (I) or their salts as claimed in any of claims 1 to 8 and formulation auxiliaries applicable in crop protection.

11. A method of controlling harmful plants or for regulating the growth of plants, which comprises applying an active amount of one or more compounds of the formula (I) or their salts as claimed in any of claims 1 to 8 to the plants, plant seeds or the area under cultivation.

12. The use of compounds of the formula (I) or their salts as claimed in any of claims 1 to 8 as herbicides or plant growth regulators.

13. The use as claimed in claim 12, wherein the compounds of the formula (I) or their salts are employed for controlling harmful plants or for regulating the growth of crops of useful plants or ornamentals.
